(19) Europäisches Patentamt
European Patent Office
Office européen des brevets

(11) Publication number: **0 049 913 B1**

# (12) EUROPEAN PATENT SPECIFICATION

(45) Date of publication of patent specification: **19.03.86**

(21) Application number: **81201040.3**

(22) Date of filing: **16.09.81**

(51) Int. Cl.$^4$: **C 07 D 233/94,** C 07 D 233/93, C 07 D 233/92, C 07 D 233/56, A 61 K 31/415, C 07 F 9/65

## (54) New ethenylimidazole derivatives, processes for their preparation and pharmaceutical compositions containing them; intermediates, useful in their preparation and a process for the preparation thereof.

(30) Priority: **17.09.80 NL 8005204**

(43) Date of publication of application:
**21.04.82 Bulletin 82/16**

(45) Publication of the grant of the patent:
**19.03.86 Bulletin 86/12**

(84) Designated Contracting States:
**AT BE CH DE FR GB IT LI LU NL SE**

(56) References cited:
**EP-A-0 003 732**
**FR-A-1 486 817**
**FR-A-2 104 344**
**FR-A-2 436 780**

(73) Proprietor: **Gist - Brocades N.V.**
**Wateringseweg 1 P.O. Box 1**
**NL-2600 MA Delft (NL)**

(72) Inventor: **Akkerboom, Piet Johannes**
**Dr. J.W. Palthelaan 48**
**NL-2712 RT Zoetermeer (NL)**
Inventor: **van der Stelt, Cornelis**
**Laurens Reaellaan 24**
**NL-2024 BG Haarlem (NL)**
Inventor: **Wegman, Bernardus**
**Vennewaard 20**
**NL-1824 KA Alkmaar (NL)**

(74) Representative: **Schmieman, Johannes Hendrik et al**
**Gist-Brocades N.V. Patents & Trademarks Department Martinus Nijhofflaan 2 P.O. Box 1**
**NL-2600 MA Delft (NL)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 913 B1

**Description**

This invention relates to new ethenylimidazole derivatives of the formula

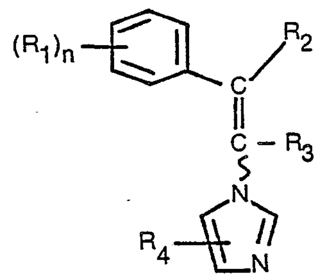

I

in which n is 0, 1 or 2, $R_1$ represents a halogen atom, a trifluoromethyl group or an alkyl or alkoxy group with at most 6 carbon atoms, the two substituents being the same or different when n is 2, $R_2$ represents an alkyl group with 1 to 6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or an alkenyl group with 2 to 4 carbon atoms and $R_4$ represents a hydrogen atom or an alkyl group with 1 to 6 carbon atoms, and their acid addition salts.

The terms "alkyl" and "alkenyl" in this specification and the accompanying claims refer to both straight and branched groups.

The new ethenylimidazole derivatives of formula I and their pharmaceutically acceptable acid addition salts are active against fungi and they can be used in combating topical and systemic disorders caused by this group of microorganisms, which includes yeasts, dermatophytes and moulds.

Compounds of formula I which in view of their pharmacological activities are preferred, are those wherein $R_1$ is chlorine, n is 1 or 2, $R_2$ is alkyl with 2 to 4 carbon atoms or cyclohexyl and $R_3$ and $R_4$ both are hydrogen atoms. More specifically preferred are the compounds in which $(R_1)_n$ is 4-chloro or 2,4-dichloro and/or $R_2$ is n-propyl, *sec*-butyl, *tert*-butyl or cyclohexyl. Particularly preferred are

1-[2-(2,4-dichlorophenyl)-3,3-dimethyl-1-butenyl]-1*H*-imidazole,
1-[2-cyclohexyl-2-(2,4-dichlorophenyl)ethenyl]-1*H*-imidazole,
1-[2-(4-chlorophenyl)-2-cyclohexylethenyl]-1*H*-imidazole,
1-[2-(2,4-dichlorophenyl)-1-pentenyl]-1*H*-imidazole,
1-[2-(4-chlorophenyl)-3-methyl-1-pentenyl]-1*H*-imidazole,
1-[2-(2,4-dichlorophenyl)-3-methyl-1-pentenyl)]-1*H*-imidazole and their acid addition salts.

The compounds are particularly effective against species of the genera *Candida* and *Trichophyton*. The compounds also show antibacterial activities, particularly against gram positive bacteria such as *Bacillus subtilis* and various species of the genus *Staphylococcus*.

Compounds that are structurally related to the compounds of formula I, are known from the literature. The French patent application no. 2,436,780 is directed to the preparation of compounds with amoebicidal and antitrichomonas activity, having the formula

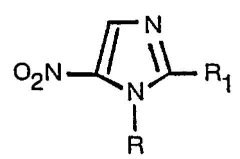

II

in which R may represent an unsaturated straight or branched hydrocarbon group, substituted with phenyl and $R_1$ may represent an alkyl group with 1 to 8 carbon atoms or a nitro group. In the compounds actually described, R is a lower alkyl group.

EP—A—003732 relates to compounds of the formula

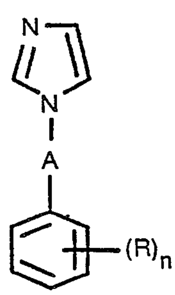

III

in which A represents a straight or branched alkylene group with 1 to 3 carbon atoms or a straight or branched alkenylene or alkinylene group with 2 or 3 carbon atoms, n is an integer (at least 1) and R represents an alkyl group with 1 to 4 carbon atoms or an alkenyl group with 2 to 4 carbon atoms. The definitions of the symbols are further subject to a number of provisos with respect to possible combinations. The compounds inhibit the synthesis of thromboxan $A_2$ and are thus active against thrombo-embolic disorders. In the compounds actually described, the imidazole nucleus is substituted in the 1-position with a benzyl or cinnamyl group.

The French patent no. 1,486,817 relates to compounds of the formula

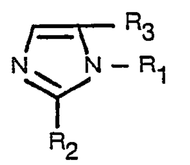

IV

The definition of $R_1$ includes a saturated or unsaturated, straight or branched alkyl chain with 1 to 13 carbon atoms, which may be substituted with a phenyl group or with a phenyl group substituted with nitro or alkyl with 1—12 carbon atoms.

$R_2$ represents hydrogen, phenyl or alkyl (1—3 C), optionally substituted with hydroxyl and $R_3$ represents hydrogen or ethyl, optionally substituted with hydroxyl or chlorine.

The compounds show a synergistic activity with insecticides of the pyrethrine, carbamate and phosphate types. The compounds actually prepared do not include phenylethenyl imidazoles.

Similar insecticidal compounds are disclosed in German Offenlegungsschrift 2750030.

According to a feature of the invention, the ethenylimidazoles of formula I are prepared by dehydrating an imidazole ethanol of the formula

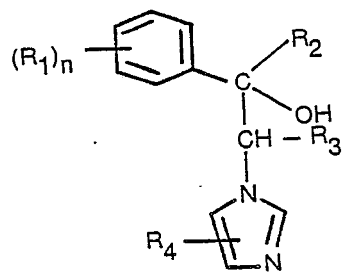

V

in which the R-symbols and n are as hereinbefore defined.

Suitable dehydrating methods include treatment with a dehydrating agent, such as thionyl chloride or hexamethyl phosphortriamide. The treatment with thionyl chloride may be carried out by heating the compound of formula V in thionyl chloride or in a mixture of thionyl chloride and another organic solvent, for instance a halogenated hydrocarbon such as chloroform, carbontetrachloride or dichloroethane. Preferably the reaction mixture is refluxed.

Alternatively the treatment with thionyl chloride may be carried out at a low temperature (e.g. between −10° and +10°C) in pyridine.

The reaction with thionyl chloride sometimes yields a compound of the formula

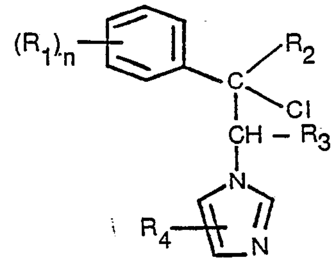

VI

in which the R-symbols and n are as hereinbefore defined, as a by-product. This compound can be converted into the desired end product of formula I by treatment with a strong base, e.g. an alkoxide with at most 6 carbon atoms, such as potassium tert. butoxide. This reaction is preferably carried out by heating the reactants at elevated temperature (e.g. 50°C) in the alcohol corresponding to the alkoxide.

When the compound of formula V is dehydrated with hexamethyl phosphortriamide, refluxing for a short period, e.g. 10 minutes, is usually sufficient to effect the dehydration.

The starting compounds of formula V may be prepared by various methods, for example by using one of the routes shown on the following reaction scheme. The route to be chosen will depend on various factors, such as the availability of starting materials and the effect of certain substituents on the desired reaction.

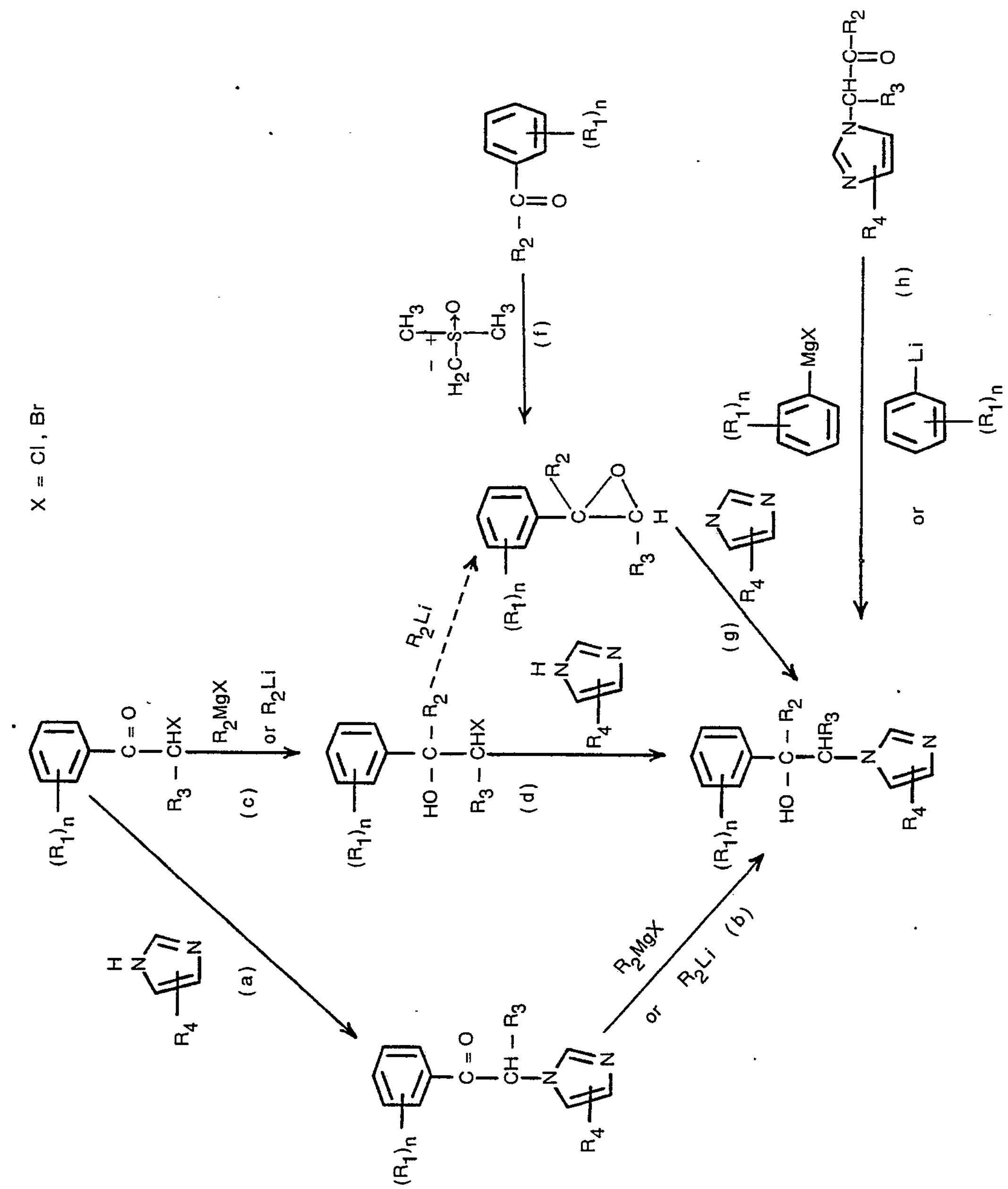
X = Cl, Br
$R_2MgX$ or $R_2Li$
(c)
(d)
(a)
$R_2MgX$ or $R_2Li$ (b)
$R_2Li$
(f)
(g)
(h)
or

Reactions (a) and (d) may be carried out by refluxing the reactants in an organic solvent, e.g. a halogenated hydrocarbon such as chloroform.

In reactions (b), (c) and (h) lithium compounds give better results than magnesium halides. The reactions with magnesium halides (b, c, h) are suitably carried out by refluxing the reactants, preferably under a nitrogen atmosphere, in an anhydrous organic solvent, such as diethyl ether or tetrahydrofuran and hydrolysing the compound obtained, for instance with a dilute ammonium chloride solution.

When a lithium compound is used, the reactions are preferably carried out under a nitrogen atmosphere in an anhydrous organic solvent such as diethyl ether or tetrahydrofuran at a temperature between 0° and −60°C. The compound obtained is then hydrolysed at room temperature e.g. with a dilute ammonium chloride solution.

In reaction (f) it is preferred to prepare dimethyloxosulfonium methylide in situ by reacting under a nitrogen atmosphere and at room temperature sodium hydride in dimethyl sulfoxide with trimethyloxosulfonium iodide. The ketone is then added to the reaction mixture and the reaction is further carried out at elevated temperature (e.g. 55—60°C).

When in reaction (c) a lithium compound is used, the alcohol obtained may in some instances further react with an excess of the lithium compound to form a phenyloxirane as obtained in reaction (f).

Reaction (g) may be carried out by heating the reactants without a solvent at a temperature of about 160°C. A number of ketone intermediates are known from the literature: see for instance E. Godefroi et al., J.Med.Chem,. 1969, *12*, 784 and the German Offenlegungsschrift no. 2838847.

According to another feature of the invention, the compounds of formula I in which $R_3$ is hydrogen, are prepared by decarboxylation of a compound of formula

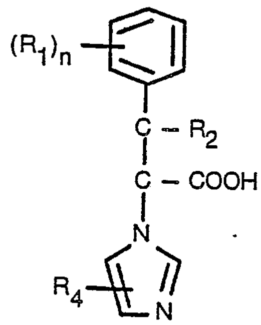

VII

in which the R-symbols and n are as hereinbefore defined. The reaction is preferably carried out by heating the compound of formula VII at a temperature between 150 and 250°C, preferably about 200°C.

The reaction results in a mixture of the E and the Z isomers of the compound of formula I, in which the E isomer is predominant. On prolonged heating, the amount of Z isomer in the mixture is increased.

The compounds of formula VII may be prepared by reacting a ketone of the formula

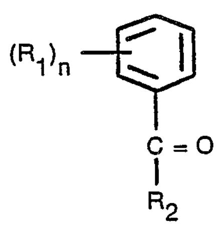

VIII

in which $R_1$, $R_2$ and n are as hereinbefore defined, with a nitrile of the formula

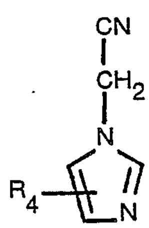

IX

in which $R_4$ is as hereinbefore defined, to obtain a compound of the formula

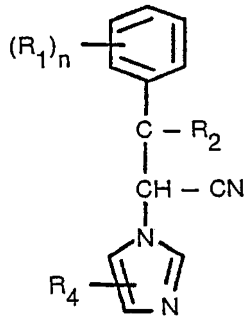

X

and hydrolyzing the latter compound. The reaction between the ketone of formula VIII and the nitrile of formula IX is preferably carried out by refluxing the reactants in an organic solvent such as tetrahydrofuran or diethyl ether in the presence of a strong base, such as lithium diisopropylamide. The nitrile of formula X may be hydrolysed directly into the acid. Alternatively the nitrile is first hydrolysed into the corresponding amide, which is then isolated and further hydrolysed into the acid of formula VII.

The conversion of the nitrile into the amide is suitably carried out by refluxing the nitrile in a mixture of a lower alkanol, e.g. ethanol and an aqueous alkali hydroxide (e.g. sodium hydroxide) solution. The conversion of the amide in the acid is suitably carried out by reacting the amide in glycol with an alkali metal hydroxide (e.g. potassium hydroxide).

According to another feature of the invention, imidazole derivatives of formula I in which $R_3$ is a hydrogen atom or an alkyl group with 1 to 6 carbon atoms and the other symbols are as defined above, are prepared by reacting an [(1*H*-imidazol-1-yl)methyl]diphenylphosphine oxide of the formula

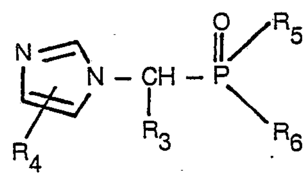

XI

in which $R_5$ and $R_6$ are the same or different and each represents an unsubstituted or substituted (e.g. with lower alkyl or lower alkoxy) phenyl group and $R_3$ and $R_4$ are as defined above, with a ketone of the formula VIII in the presence of an alkyl lithium and subsequent decomposition of the product obtained with water. The reaction between the compounds of formulae XI and VIII is preferably carried out in an inert organic solvent such as tetrahydrofuran, diethyl ether, 1,2-dimethoxyethane or dioxane at a temperature between −40 and 0°C, more particularly between −30 and −20°C.

The compounds of formula XI are new and as such constitute another feature of the invention, compounds in which $R_5$ and $R_6$ are unsubstituted phenyl being preferred.

According to a further feature of the invention the compounds of formula XI are prepared by reacting a diphenylphosphine-oxide of the formula

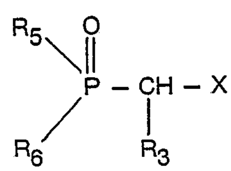

XII

in which X is a chlorine or bromine atom and $R_3$, $R_5$ and $R_6$ are as defined above, with an imidazole derivative of the formula

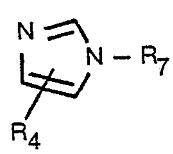

XIII

in which $R_7$ is a sodium, potassium or lithium atom. The reaction is preferably carried out in a polar organic solvent, such as dimethylformamide, dimethylacetamide, 1-methylpyrrolidone, dimethylsulphoxide, hexamethylphosphortriamide or acetonitrile, at a temperature between 50 and 100°C. The diphenylphosphine-

oxides of formula XII may be prepared as described in J.Organomet.Chem., *94*, 327 (1975).

The preparation of the compounds of the invention is illustrated by the following examples. The structures of the prepared compounds were confirmed by their NMR and mass spectra. The purity of the compounds was verified by titration and thin layer chromatography.

Example 1

(a) A solution of 98 g (0.42 mole) of 2,4'-dichloroacetophenone was added dropwise with stirring to a refluxing solution of 69 g (0.84 mole) of 2-methylimidazole and 84 g (0.84 mole) of triethylamine in 300 ml of chloroform. The reaction mixture was refluxed for another 4 hours and it was then kept at room temperature for about 60 hours. Water was then added to the reaction mixture and the solid substance, which dissolved badly in chloroform, was filtered off (I). The organic layer of the filtrate was separated off, washed four times with water and concentrated by evaporation of the solvent. The residue was crystallized from a mixture of ethanol and petroleum ether (boiling range 40—60°C) (II). Thin layer chromatography showed that the products I and II were identical.

18 g of 1-(4-chlorophenyl)-2-(2-methyl-1*H*-imidazol-1-yl)ethanone were obtained. Melting point 215°C.

(b) *Tert* butylmagnesium chloride was prepared by adding dropwise, in the course of about 5 hours, and under a nitrogen atmosphere 13.9 g (0.15 mole) of tert. butyl chloride to 4 g of magnesium in 200 ml of anhydrous diethyl ether. A suspension of 11.7 g (0.05 mole) of 1-(4-chlorophenyl)-2-(2-methyl-1*H*-imidazol-1-yl)ethanone in 200 ml of anhydrous tetrahydrofuran was then slowly added while the diethyl ether was simultaneously distilled off. The mixture was subsequently refluxed for 18 hours. The reaction mixture was then cooled to 0°C and decomposed with a dilute ammonium chloride solution. The organic layer was separated off and the solvent was distilled off. The residue was dissolved in acetone and a solution of hydrogen chloride in diethyl ether was added until the reaction mixture was acidic. The precipitate formed was crystallized from acetone.

α-(4-Chlorophenyl)-α-(1,1-dimethylethyl)-2-methyl-1*H*-imidazole-1-ethanol hydrochloride was obtained. Melting point 239°C.

(c) 3.8 g of the compound obtained under (b) and 75 ml of thionyl chloride were refluxed for three hours with stirring. The residue was dissolved in dichloromethane and then diethyl ether was added. The solid substance formed was filtered off and dried in vacuo at about 60°C.

1-[2-(4-Chlorophenyl)-3,3-dimethyl-1-butenyl]-2-methyl-1*H*-imidazole hydrochloride was obtained. Melting point 261°C.

Example 2

(a) 100 ml of a 15% butyl lithium solution in hexane were slowly and dropwise added to a solution of 17 g (0.1 mole) of 4-bromotoluene in 30 ml of diethyl ether, which made the temperature rise from 20 to 40°C. The mixture was stirred for 1½ hrs. and then 80 ml of hexane were added and subsequently a solution of 8.5 g (0.05 mole) of 1-(1*H*-imidazol-1-yl)-3,3-dimethyl-2-butanone (described in British Patent Specification no. 1,535,777) in 10 ml of diethyl ether. An oily precipitate was formed, which partly dissolved after some time. The mixture was stirred for two hours at room temperature and then the reaction mixture was decomposed by adding a small amount of water. On acidifying the mixture with concentrated hydrochloric acid, the hydrochloride of α-(1,1-dimethylethyl)-α-(4-methylphenyl)-1*H*-imidazole-1-ethanole precipitated. The salt was dried in vacuo at about 60°C. Melting point >250°C.

(b) A mixture of 2.9 g (0.01 mole) of the compound obtained under a) and 20 ml of thionyl chloride were refluxed for 3 hours. After evaporation of the liquid, 4N ammonia was added to the residue. The mixture was then extracted with dichloromethane. The solvent was distilled off and the crude residue was passed over a silica column with a mixture of chloroform, ethyl acetate and triethylamine (30:30:10) as the eluent. The base obtained was dissolved in ethyl acetate and acidified with concentrated nitric acid. The solvent was distilled off and the residue was dissolved in a mixture of acetone and diethyl ether, from which 1-[3,3-dimethyl-2-(4-methylphenyl-1-butenyl]-1*H*-imidazole nitrate precipitate. The compound was filtered off and dried in vacuo at about 60°C. Melting point 157°C.

Example 3

a) 100 ml of a 15% butyl lithium solution in hexane were added slowly and dropwise to a solution of 18.7 g (0.1 mole) of 1-bromo-4-methoxybenzene in 40 ml of diethyl ether. The mixture was stirred for half an hour and then a solution of 1-(1*H*-imidazol-1-yl)-3,3-dimethyl-2-butanone in 10 ml of diethyl ether was added. The reaction mixture was stirred for another two hours and it was then decomposed with water and acidified with concentrated hydrochloric acid. The precipitate was filtered off, washed with a small amount of cold acetone and dried in vacuo at about 60°C. The NMR spectrum showed that the product was a mixture of α-(1,1-dimethylethyl)-α-(4-methoxyphenyl)-1*H*-imidazole-1-ethanol hydrochloride (A) and α-(5-bromo-2-methoxyphenyl)-α-(1,1-dimethylethyl)-1*H*-imidazole-1-ethanol hydrochloride (B). Crystallization from a mixture of little methanol and a large amount of ethyl acetate yielded compound B. The mother lye was concentrated by evaporating the solvents and the residue was crystallised from a mixture of 2-propanol and diethyl ether, which yielded compound A (still slightly contaminated with B). Melting point 239°C.

b) A mixture of 1.5 g (0.005 mole) of compound A (described under a) and 30 ml of thionyl chloride was

refluxed for three hours. The liquid was distilled off and the residue was taken up in a mixture of 4N ammonia and dichloromethane. The organic layer was dried on sodium sulphate and the solvent was distilled off. The residue was purified over a silica gel column with a mixture of chloroform, ethyl acetate and 25% ammonia (50:50:1) as the eluent.

1-[2-(4-methoxyphenyl)-3,3-dimethyl-1-butenyl]-1*H*-imidazole was isolated. The compound was dissolved in ethyl acetate and the solution was acidified with concentrated nitric acid. On addition of diethyl ether, the nitrate crystallised. The salt was filtered off and dried in vacuo at about 60°C. Melting point 157°C.

Example 4

A mixture of 1.9 g (0.005 mole) of α-(5-bromo-2-methoxyphenyl)-α-(1,1-dimethylethyl)-1*H*-imidazole-1-ethanol hydrochloride (compound B in Example 3a) and 20 ml of thionyl chloride was refluxed for 3 hours. The excess thionyl chloride was distilled off and 4N ammonia was added to the residue. The aqueous phase was extracted with dichloromethane, the extract was dried on sodium sulphate and the solvent was distilled off. The oily residue was purified over a silica gel column with a mixture of chloroform, ethyl acetate and 25% ammonia (50:50:1) as the eluent. The substance obtained was dissolved in ethyl acetate and acidified with concentrated nitric acid. On addition of diethyl ether, 1-[2-(5-bromo-2-methoxyphenyl)-3,3-dimethyl-1-butenyl]-1*H*-imidazole nitrate precipitated. The salt was filtered off and dried in vacuo at about 60°C. Melting point 181°C.

Example 5

a) 2.2 g (0.055 mole) of 60% sodium hydride in oil were washed under a nitrogen atmosphere with two portions of 50 ml of petroleum ether (boiling point 40—60°C). After removal of the petroleum ether 60 ml of anhydrous dimethyl sulfoxide were added and subsequently 12.7 g of trimethyloxosulfonium iodide were added with stirring in the course of 15 minutes. The reaction mixture — containing dimethyloxosulfonium methylide (see Org.Synth.Coll.Vol. V. 755 (1973)) — was stirred for another half hour at room temperature and then a solution of 12 g (0.05 mole) of 1-(3,4-dichlorophenyl)-2,2-dimethyl-1-propanone in 10 ml of dimethyl sulfoxide was added dropwise in the course of 5 minutes.

The reaction mixture was stirred for half an hour at room temperature and it was then heated for half an hour at 55—60°C. The mixture was then cooled and poured into water. The mixture was extracted with diethyl ether, the extract was washed five times with water and dried on sodium sulphate and the solvent was distilled off. The oily product, consisting mainly of 2-(3,4-dichlorophenyl)-2-(1,1-dimethylethyl)oxirane, was used without further purification in the next reaction step.

b) 10 g of the oil obtained under a) and 8.2 g of 1*H*-imidazole were heated for 1½ hours at 160°C with stirring. The reaction mixture was cooled and water and dichloromethane were added. The organic layer was separated off and washed with water and the solvent was evaporated. The residue was dissolved in acetone and the solution was acidified with hydrogen chloride in diethyl ether. The precipitated α-(3,4-dichlorophenyl)-α-(1,1-dimethylethyl)-1*H*-imidazole-1-ethanol hydrochloride was filtered off and dried in vacuo at about 60°C. Melting point 267°C.

c) A mixture of 10 g of α-(3,4-dichlorophenyl)-α-(1,1-dimethylethyl)-1*H*-imidazole-1-ethanol hydrochloride and 200 ml of thionyl chloride was refluxed for 6 hours with stirring. The liquid was distilled off and 4N ammonia was added to the residue and the mixture was then extracted with dichloromethane. The organic layer was concentrated by evaporation of the solvent and the residue was purified over a silica gel column with a mixture of chloroform, ethyl acetate and ammonia (50:50:1) as the eluent. The base obtained was dissolved in acetone and the solution was acidified with hydrogen chloride in diethyl ether. The precipitated 1-[2-(3,4-dichlorophenyl)-3,3-dimethyl-1-butenyl]-1-*H*-imidazole hydrochloride was filtered off and dried in vacuo at about 60°C. Melting point 266°C.

Example 6

a) A Grignard compound was prepared from 5.4 g (0.06 mole) of *tert* butylchloride and 1.5 g (0.06 at) of magnesium in 100 ml of anhydrous tetrahydrofuran. The reaction mixture was refluxed for one hour and then a solution of 8.8 g (0.04 mole) of 1-(4-chlorophenyl)-2-(1*H*-imidazol-1-yl)ethanone (E. Godefroi et al., J.Med.Chem. *12*, 784 (1969)) in 100 ml of anhydrous tetrahydrofuran was added dropwise. The solution was refluxed for two hours and then cooled and poured into an aqueous ammonium chloride solution. The organic layer was separated off and dried on sodium sulphate and the solvent was distilled off. The residue was dissolved in diethyl ether and the solution was acidified with concentrated hydrochloric acid. The precipitate was filtered off, washed with acetone and diethyl ether and dried in vacuo at about 60°C.

α-(4-Chlorophenyl)-α-(1,1-dimethylethyl)-1*H*-imidazole-1-ethanol hydrochloride was obtained. Melting point 284°C.

b) 13.5 g (0.042 mole) of α-(4-chlorophenyl)-α-(1,1-dimethylethyl)-1*H*-imidazole-1-ethanol hydrochloride were suspended in 150 ml of chloroform and the suspension was saturated with hydrogen chloride gas. The liquid was distilled off and 150 ml of thionyl chloride were added to the residue. The suspension obtained was refluxed for two hours with stirring. The liquid was distilled off and the residue was extracted with chloroform and water. The organic phase was washed successively with water and with dilute ammonia and dried on sodium sulphate and the solvent was distilled off. The resulting oil was dissolved in a mixture of acetone and diethyl ether (1:1) and acidified with concentrated nitric acid. The

precipitate formed was filtered off and crystallised from 2-propanol.

1-[2-(4-Chlorophenyl)-3,3-dimethyl-1-butenyl]-1*H*-imidazole nitrate was obtained. Melting point 170°C.

Example 7

a) A Grignard compound, prepared from 4.8 g (0.2 at) of magnesium and 31 g (0.2 mole) of hexyl bromide in 120 ml of diethyl ether was refluxed for two hours. The ether was distilled off and tetrahydrofuran was simultaneously added. The reaction mixture was then cooled to 0°C on which some solid was formed. A solution of 18 g (0.1 mole) of 2-(1*H*-imidazol-1-yl)-1-phenylethanone (E. Godefroi et al., J.Med.Chem. *12*, 784 (1969)) in 150 ml of tetrahydrofuran was added dropwise with stirring at 0—5°C, after which the mixture was stirred for two hours under cooling and was then kept overnight at room temperature. The mixture was then decomposed with a saturated ammonium chloride solution and the organic layer was separated off and dried on sodium sulphate and the liquid was distilled off. The residue was boiled with 100 ml of ethyl acetate and the solid was filtered off after cooling. The filtrate was concentrated by evaporation of the solvent and the residue was purified over a silica gel column with a mixture of chloroform, ethyl acetate and 25% ammonia (50:50:1.5) as the eluent.

α-Hexyl-α-phenyl-1*H*-imidazole-1-ethanol was obtained. Melting point 139°C.

b) Hydrogen chloride gas was passed through a solution of 4.0 g (0.0147 mole) of α-hexyl-α-phenyl-1*H*-imidazole-1-ethanol in chloroform to saturation. After evaporation of the solvent, the residue was taken up in 60 ml of thionyl chloride, after which the clear solution was refluxed with stirring for 3 hours. The liquid was distilled off and the residue was taken up in chloroform and an aqueous solution of sodium bicarbonate. The organic layer was separated off and dried on sodium sulphate and the solvent was distilled off. The residue was purified over a silica gel column with a mixture of chloroform, ethyl acetate and 25% ammonia (50:50:2) as the eluent. 1-(2-Phenyl-1-octenyl)-1*H*-imidazole was isolated as an oil.

Example 8

a) Under a nitrogen atmosphere and at −60°C 80 ml of a 15% solution of butyl lithium in hexane were added dropwise with stirring to a suspension of 14 g (0.064 mole) of 1-(4-chlorophenyl-2-1*H*-imidazol-1-yl)ethanone in 250 ml of anhydrous diethyl ether. The mixture was stirred for one hour at −60°C and the temperature was then raised to −10°C, after which 100 ml of tetrahydrofuran were added. The reaction mixture was stirred at −10°C for another 4 hours and subsequently decomposed with an aqueous ammonium chloride solution. The organic layer was separated off, dried on sodium sulphate and the liquid was evaporated. The residue was taken up in diethyl ether and the slowly crystallising solid was filtered off. The filtrate was concentrated by evaporation of the solvent and the residue was purified over a silica gel column with a mixture of chloroform, ethyl acetate and 25% ammonia (50:50:1) as the eluent. The appropriate fractions were combined and the solvent was distilled off. The residue crystallised on addition of a small amount of diethyl ether. The crude α-butyl-α-(4-chlorophenyl)-1*H*-imidazole-1-ethanol thus obtained was used in the next reaction step.

b) A solution of 5.0 g (0.018 mole) of crude α-butyl-α-(4-chlorophenyl-1*H*-imidazole-1-ethanol in 100 ml of chloroform was saturated with hydrogen chloride gas. The solvent was distilled off and the residue was dissolved in 100 ml of thionyl chloride, after which the clear solution was refluxed for two hours with stirring. The solvent was evaporated and the residue was extracted with ethyl acetate and 4N ammonia. The organic layer was separated off and dried on sodium sulphate and the solvent was distilled off. The resulting oil was purified over a silica gel column with a mixture of chloroform, ethyl acetate and 25% ammonia (50:50:1) as the eluent.

1-[2-(4-Chlorophenyl)-1-hexenyl]-1*H*-imidazole was isolated. It was converted into the oxalate, which melted at 145.5°C.

Example 9

a) A Grignard compound was prepared from 6.6 g (0.08 mole) of *tert* butylchloride and 1.95 g (0.08 at) of magnesium in 100 ml of anhydrous tetrahydrofuran. The mixture was refluxed for one hour and then a solution of 12.7 g (0.05 mole) of 1-(2,4-dichlorophenyl)-2-1*H*-imidazol-1-yl-ethanone in 100 ml of anhydrous tetrahydrofuran was added dropwise with stirring. The solution was refluxed for two hours and it was then cooled and poured into an aqueous ammonium chloride solution. The organic layer was separated off and dried over sodium sulphate. The solvent was then distilled off and the residue was taken up in acetone and acidified with concentrated nitric acid. Addition of diethyl ether made α-(2,4-dichlorophenyl)-α-(1,1,-dimethyl)-1*H*-imidazole-1-ethanol nitrate precipitate. The salt was filtered off, washed with diethyl ether and dried in vacuo at about 60°C. Melting point 145°C.

b) The nitrate obtained under a) was converted into its hydrochloride and 12.9 g (0.037 mole) of the salt was suspended in 250 ml of chloroform. The suspension was saturated with hydrogen chloride gas and then the liquid was evaporated. The residue was dissolved in 125 ml of thionyl chloride and the solution was refluxed for three hours with stirring. The liquid was then distilled off and the residue was extracted with an aqueous sodium bicarbonate solution and with chloroform. The organic layer was separated off, dried on sodium sulphate and finally concentrated by evaporation of the solvent. The resulting oil was taken up in ethyl acetate and acidified with concentrated nitric acid. The precipitate formed, consisting of 1-[2-(2,4-dichlorophenyl)-3,3-dimethyl-1-butenyl]-1*H*-imidazole nitrate, was filtered off, washed with a

mixture of ethyl acetate and diethyl ether and dried in vacuo at about 60°C. Melting point 188°C.

Example 10

a) A solution of 7.4 g (0.04 mole) of 2-(1*H*-imidazol-1-yl)-1-phenylethanone in 100 ml of anhydrous tetrahydrofuran was added dropwise with stirring to a Grignard compound prepared from 5.55 g (0.06 mole) of tert. butyl chloride and 1.5 g (0.06 at) of magnesium in 100 ml of anhydrous tetrahydrofuran. The solution was refluxed for two hours, cooled and poured into an aqueous ammonium chloride solution. The organic layer was separated off, and dried on sodium sulphate and the solvent was distilled off. A solution of hydrogen chloride in 2-propanol was added to the residue and the precipitate, consisting of α-(1,1-dimethylethyl)-α-phenyl-1*H*-imidazole-1-ethanol hydrochloride, was filtered off and dried in vacuo at about 60°C.

b) A suspension of 4.3 g (0.015 mole) of α-(1,1-dimethylethyl)-α-phenyl-1*H*-imidazole-1-ethanol hydrochloride in 100 ml of chloroform was saturated with hydrogen chloride gas. The solvent was then distilled off and the residue was taken up in 100 ml of chloroform and 50 ml of thionyl chloride. The solution was refluxed for two hours, after which the liquid was distilled off. The residue obtained was treated with chloroform and aqueous sodium bicarbonate solution. The organic layer was separated off and dried on sodium sulphate and the solvent was distilled off. The residue was dissolved in ethyl acetate and acidified with concentrated nitric acid. The precipitate of 1-(3,3-dimethyl-2-phenyl-1-butenyl)-1*H*-imidazole nitrate was filtered off, washed with diethyl ether and dried in vacuo at about 60°C. Melting point 188°C.

Example 11

a) 46 ml of thionyl chloride were added dropwise at 0°C to a suspension of 69 g (0.2 mole) of α-(2,4-dichlorophenyl)-α-(1,1-dimethylethyl)-1*H*-imidazole-1-ethanol nitrate (see Example 9a) in 328 ml of pyridine. The mixture was stirred for one hour at 0°C, the temperature was then raised to room temperature and stirring was continued for another hour. The mixture was then poured in ice and extracted twice with ethyl acetate. The extract was washed six times with water, dried on sodium sulphate and concentrated by evaporation of the solvent. The residue was dissolved in 2-propanol and the solution was acidified with a solution of hydrogen chloride gas in 2-propanol, after which the same volume of a mixture of ethyl acetate and diethyl ether (1:1) was added. After cooling, the solid was filtered off and washed with ethyl acetate and diethyl ether. The 1-[2-(2,4-dichlorophenyl)-3,3-dimethyl-1-butenyl]-1*H*-imidazole hydrochloride obtained was crystallised from a mixture of ethyl acetate and diethyl ether (1:1). Melting point 254°C.

b) 6.6 g (0.02 mole) of the hydrochloride obtained under a) were dissolved in chloroform and 2N sodium hydroxide solution was added to liberate the base. The organic layer was separated off and the solvent was evaporated. The residue was dissolved in ethyl acetate, the solution was dried on sodium sulphate and the solvent was distilled off. The residue was crystallised from petroleum ether (boiling point 40—60°C). The base obtained melted at 88°C.

Example 12

a) A Grignard compound was prepared from 35 g (0.3 mole) of cyclohexyl chloride and 7.3 g (0.3 at) of magnesium in diethyl ether. The ether was then distilled off, while tetrahydrofuran was simultaneously added. A solution of 25.2 g (0.1 mole) of 1-(2,4-dichlorophenyl)-2-(1*H*-imidazole-1-yl)ethanone in 200 ml of tetrahydrofuran was subsequently added dropwise with stirring to the refluxing solution. The mixture was refluxed for two hours and was then decomposed with aqueous ammonium chloride solution, after which the organic layer was separated off, dried on sodium sulphate and concentrated by evaporation of the solvent. The residue was purified over a silica gel column with a mixture of chloroform, ethyl acetate and 25% ammonia (50:50:1) as the eluent. α-Cyclohexyl-α-(2,4-dichlorophenyl)-1*H*-imidazole-1-ethanol was obtained.

b) At 0°C 3 ml of thionyl chloride were added dropwise with stirring to a solution of 4.0 g (0.0118 mole) of α-cyclohexyl-α-(2,4-dichlorophenyl)-1*H*-imidazol-1-ethanol in 50 ml of pyridine. The mixture was stirred for another two hours and it was then poured into a mixture of ice and water. The mixture was then extracted with ethyl acetate. The organic layer was separated off, dried on sodium sulphate and concentrated by evaporation of the solvent. The residue was purified over a silica gel column with a mixture of chloroform, ethyl acetate and 25% ammonia (50:50:1) as the eluent. The appropriate fractions were combined and the solvent was distilled off. The resulting oil was dissolved in diethyl ether and the solution obtained was acidified with a solution of hydrogen chloride in 2-propanol, which yielded a precipitate of 1-[2-cyclohexyl-2-(2,4-dichlorophenyl)ethenyl]-1*H*-imidazole hydrochloride. The salt was filtered off and dried in vacuo at about 60°C. Melting point 265.5°C.

Example 13

a) 40 ml of thionyl chloride were added dropwise with stirring at 0°C to a solution of 57.6 g (0.18 mole) of α-(4-chlorophenyl)-α-(1,1-dimethylethyl)-1*H*-imidazol-1-ethanol hydrochloride (see Example 6a) in 300 ml of pyridine. The mixture was stirred for half an hour at 0°C, the temperature was raised to room temperature and the mixture was then poured into ice. The mixture obtained was extracted with diethyl ether, the extract was washed six times with water and dried on sodium sulphate and the solvent was

distilled off. The residue was crystallised from a mixture of diethyl ether and petroleum ether (boiling point 40—60°C).

1-[2-(4-Chlorophenyl)-3,3-dimethyl-1-butenyl]-1*H*-imidazole was obtained. Melting point 101°C.

b) A part of the product was converted into the hydrochloride by dissolving it in 2-propanol and adding a solution of hydrogen chloride in 2-propanol. The precipitated salt was filtered off, crystallised from 2-propanol and dried in vacuo at about 60°C. The salt decomposed on heating.

Example 14

a) 11 g (0.05 mole) of 1-(2-chlorophenyl)-2-(1*H*-imidazol-1-yl)ethanone in 125 ml of tetrahydrofuran were added dropwise to 40 ml of a refluxing solution of 25% tert. butyl magnesium chloride in diethyl ether. The reaction mixture was refluxed for another 2.5 hours and it was then cooled and decomposed by adding an aqueous ammonium chloride solution. The organic layer was separated off, dried on sodium sulphate and concentrated by evaporation of the solvent. The oily residue was purified over a silica gel column with a mixture of chloroform, ethyl acetate and 25% ammonia (50:50:1) as the eluent. The substance obtained solidified and it was washed with diethyl ether.

α-(2-Chlorophenyl)-α-(1,1-dimethylethyl)-1*H*-imidazole-1-ethanol was obtained. Melting point 157°C.

b) Under a nitrogen atmosphere and at 0°C, 2 ml of thionyl chloride were added dropwise with stirring to a solution of 2.2. g of α-(2-chlorophenyl)-α-(1,1-dimethylethyl) 1*H*-imidazole-1-ethanol in 20 ml of pyridine. The reaction mixture was stirred for 1½ hours at 0°C and then for one hour at 20°C. It was subsequently poured into ice and the mixture obtained was extracted with ethyl acetate. The extract was dried on sodium sulphate and the solvent was distilled off. The resulting oily residue was dissolved in diethyl ether and a solution of hydrogen chloride in 2-propanol was added to the solution, on which 1-[2-(chlorophenyl)-3,3-dimethyl-1-butenyl]-1*H*-imidazole hydrochloride precipitated. The salt was crystallised from a mixture of acetone and diethyl ether and dried in vacuo at about 60°C. Melting point 210°C.

Example 15

a) A solution of 27 g (0.25 mole) of ethyl bromide in 30 ml of diethyl ether was added with vigorous stirring at −10°C to 3.5 g (0.5 at) of lithium wire in 90 ml of anhydrous diethyl ether. The mixture was stirred for half an hour at −10°C and the solution of ethyl lithium thus obtained was added dropwise at −70°C to a mixture of 44.7 g (0.2 mole) of 2,2′,4′-trichloroacetophenone in 250 ml of diethyl ether. The reaction mixture was stirred for one hour at −50°C and then for three hours at −10°C and it was subsequently decomposed with water. The organic layer was separated off, washed with water and the solvent was distilled off. The residue was distilled, which yielded 2-(2,4-dichlorophenyl)-2-ethyloxirane. Boiling point 85—90°C (0.1 mm Hg).

b) A mixture of 24 g (0.11 mole) of 2-(2,4-dichlorophenyl)-2-ethyloxirane and 22.5 g (0.3 mole) of 1*H*-imidazole was heated at 160°C for two hours with stirring. The reaction mixture was poured in water and extracted with a mixture of diethyl ether and dichloromethane (4:1). The organic layer was separated off and washed with water and the solvent was distilled off. The residue was dissolved in acetone and the solution was acidified with a solution of hydrogen chloride in diethyl ether. The precipitated α-(2,4-dichlorophenyl)-α-ethyl-1*H*-imidazole-1-ethanol hydrochloride was filtered off, washed with acetone and dried in vacuo at 60°C. Melting point 202°C.

c) 5 ml of thionyl chloride were added dropwise at 0°C with stirring to a solution of 6 g of α-(2,4-dichlorophenyl)-α-ethyl-1*H*-imidazole-1-ethanol hydrochloride in 30 ml of anhydrous pyridine. The mixture was stirred for one hour at 0°C, it was then allowed to attain room temperature and poured in ice water. The oily precipitate was dissolved in diethyl ether, the ethereal layer was separated off, washed 5 times with water and dried on sodium sulphate and the ether was distilled off. The residue was dissolved in acetone and the solution was acidified with a solution of hydrogen chloride in diethyl ether. The precipitated 1-[2-(2,4-dichlorophenyl)-1-butenyl]-1*H*-imidazole hydrochloride was filtered off and dried in vacuo at about 60°C. Melting point 165°C.

Example 16

a) A propyl lithium solution was prepared at −10°C from 5.25 g (0.75 at) lithium wire and 46 g (0.375 mole) of n-propyl bromide in 200 ml of diethyl ether. The solution was added dropwise at −60°C to a mixture of 44.7 g of 2-chloro-1-(2,4-dichlorophenyl)ethanone and 250 ml of diethyl ether. After having reacted for one hour at −60°C, the reaction mixture was allowed to attain room temperature and it was then poured into water. The organic layer was separated off and dried on sodium sulphate and the solvent was evaporated. The residue was distilled.

2,4-dichloro-α-(chloromethyl)-α-propylbenzenemethanol was obtained. Boiling point 110—120°C/0.1 mm Hg.

b) A mixture of 25 g of the crude product obtained under a, and 34 g of 1*H*-imidazole was heated for two hours at 140°C. The reaction mixture was cooled to 80°C and it was then poured into water and the mixture was extracted with diethyl ether. During the extraction a solid substance crystallised. The substance was filtered off, washed with water and diethyl ether and dried in vacuo at about 60°C.

α-(2,4-Dichlorophenyl)-α-propyl-1*H*-imidazole-1-ethanol was obtained. Melting point 155°C.

c) 2.5 ml of thionyl chloride were slowly added dropwise with stirring at 0°C to a mixture of 5 g of α-

(2,4-dichlorophenyl)-α-propyl-1H-imidazole-1-ethanol and 25 ml of pyridine. The mixture was stirred for two hours at 0°C and it was then poured into ice water. The mixture was extracted with diethyl ether, the extract was washed five times with water, and dried on sodium sulphate and the solvent was distilled off. The residue was dissolved in acetone and a solution of hydrogen chloride in diethyl ether was added to acidic reaction. The compound first crystallising was 1-[2-chloro-2-(2,4-dichlorophenyl)pentyl]-1*H*-imidazole hydrochloride. From the mother lye, 1-[2-(2,4-dichlorophenyl)-1-pentenyl]-1*H*-imidazol hydrochloride could be obtained. Melting point 174°C.

Example 17

a) A Grignard compound was prepared from 8 g (0.33 at) of magnesium and 37 g (0.3 mole) of isopropyl bromide in 200 ml of anhydrous diethyl ether. The ether was evaporated while an equal amount of tetrahydrofuran was simultaneously added. The solution obtained was added dropwise with stirring to a refluxing solution of 20 g (0.08 mole) of 1-(2,4-dichlorophenyl)-2-(1*H*-imidazol-1-yl)ethanone in 50 ml of tetrahydrofuran. The reaction mixture was refluxed for one hour and it was then cooled to 0°C and decomposed with a saturated aqueous ammonium chloride solution. The organic layer was separated off, the solvent was evaporated and the residue was purified by column chromatography (silicagel; chloroform/ethyl acetate/25% ammonia 50:50:1). The base obtained was converted into α-(1-methylethyl)-α-(2,4-dichlorophenyl)-1*H*-imidazole-1-ethanol hydrochloride. Melting point 290°C.

b) Following the procedure described in Example 15c, but substituting α-(1-methylethyl)-α-(2,4-dichlorophenyl)-1*H*-imidazole-1-ethanol hydrochloride for the α-(2,4-dichlorophenyl)-α-ethyl-1*H*-imidazole-1-ethanol hydrochloride, there was obtained 1-[2-(2,4-dichlorophenyl)-3-methyl-1-butenyl]-1*H*-imidazole hydrochloride. Melting point 219°C.

Example 18

a) 120 ml (0.2 mole) of a 15% butyl lithium solution in hexane were added dropwise with stirring at −60°C and under a nitrogen atmosphere to a suspension of 44.7 g (0.2 mole) of 2,2′,4′-trichloroacetophenone in 250 ml of anhydrous diethyl ether. The reaction mixture was stirred for one hour at −55°C and for three hours at −10°C and it was then decomposed with a saturated aqueous ammonium chloride solution. The organic layer was separated off, dried on sodium sulphate and concentrated by evaporation of the liquid. The residue was distilled. α-Butyl-2,4-dichloro-α-(chloromethyl)benzenemethanol was obtained.

b) A mixture of 17 g (0.25 mole) of imidazole and 14.1 g (0.05 mole) of α-butyl-2,4-dichloro-α-(chloromethyl)benzenemethanol was heated for three hours at 120°C with stirring. The mixture was cooled and it was then extracted with chloroform and water. The chloroform layer was separated off and the solvent was distilled off. The residue was dissolved in diethyl ether and the solution was extracted a few times with water and then dried and concentrated by evaporating the ether. The residue was dissolved in diethyl ether and the solution was acidified with concentrated nitric acid. The precipitated α-butyl-α-(2,4-dichlorophenyl)-1*H*-imidazol-1-ethanol nitrate was filtered off and dried in vacuo at about 60°C.

c) The salt obtained in Example 18b was converted into the free base and a solution of 4.8 g (0.015 mole) of the base in 100 ml of chloroform was saturated with hydrogen chloride gas. The chloroform was distilled off, the residue was taken up in thionyl chloride and the solution obtained was refluxed for two hours. The liquid was then distilled off and the residue was extracted with chloroform and 4N ammonia. The organic layer was dried on sodium sulphate and the solvent was distilled off. To the residue 100 ml of *tert* butanol and 7 g of potassium *tert* butoxide were added and the mixture was then heated to 50°C with stirring. The solvent was distilled off and the residue was extracted with ethyl acetate and water. The organic layer was separated off and dried on sodium sulphate and the solvent was distilled off. The residue was dissolved in ethyl acetate and the solution was acidified with a solution of oxalic acid in diethyl ether. The precipitated 1-[2-(2,4-dichlorophenyl)-1-hexenyl]-1*H*-imidazole ethanedioate (1:1) was filtered off, washed with diethyl ether and dried in vacuo at about 60°C. Melting point 151°C.

Example 19

a) A Grignard solution was prepared from 12 g (0.1 mole) of 1-chloro-3,3-dimethylbutane and 2.3 g (0.1 at) of magnesium in 100 ml of anhydrous diethyl ether. The solution was refluxed with stirring for one hour and then the ether was distilled off, while an equal amount of tetrahydrofuran was simultaneously added. 13.0 g (0.07 mole) of 2-(1*H*-imidazol-1-yl)-1-phenylethanone in 200 ml of tetrahydrofuran were added dropwise to the refluxing solution. The reaction mixture was refluxed for two hours and it was then poured into an aqueous ammonium chloride solution. The organic layer was separated off and dried on sodium sulphate and the solvent was distilled off. The residue was purified over a silica gel column with a mixture of chloroform, ethyl acetate and 25% ammonia (50:50:2) as the eluent. α-(3,3-Dimethylbutyl)-α-phenyl-1*H*-imidazole-1-ethanol was obtained. Melting point 190°C.

b) A solution of 7.2 g (0.026 mole) of α-(3,3-dimethylbutyl)-α-phenyl-1*H*-imidazole-1-ethanol in 150 ml of chloroform was saturated with hydrogen chloride gas. The solvent was distilled off, the residue was dissolved in 100 ml of thionyl chloride and the solution was refluxed for three hours with stirring. The solvent was distilled off and the residue was extracted with ethyl acetate and 4N ammonia. The organic layer was dried on sodium sulphate and the solvent was distilled off. The residue was taken up in 50 ml of

*tert* butanol, 8 g of potassium *tert* butoxide were added and the mixture was heated for three hours at 50°C with stirring. The solvent was then distilled off and the residue was extracted with ethyl acetate and water. The organic layer was separated off and dried on sodium sulphate and the solvent was distilled off. The oily residue was dissolved in 2-propanol and the solution was acidified with ethereal hydrogen chloride. The precipitate was filtered off and dried in vacuo at about 60°C. It consisted of a mixture (1:1) of the E and Z isomers of 1-(5,5-dimethyl-2-phenyl-1-hexenyl)-1*H*-imidazole hydrochloride. Melting point 170°C.

The filtrate was concentrated by evaporation of the solvent and ethyl acetate and diethyl ether were added to the residue. The solid obtained was filtered off, boiled with ethyl acetate, filtered off again and dried in vacuo at about 60°C. It consisted of the same isomer with the ratio Z:E = 3:1.

Example 20

a) A Grignard compound was prepared from 3.6 g (0.15 at) of magnesium and 16 g (0.13 mole) of 1-chloro-3,3-dimethylbutane in diethyl ether and the solution was refluxed for one hour. A solution of 11 g (0.05 mole) of 1-(4-chlorophenyl)-2-(1*H*-imidazol-2-yl)ethanone in tetrahydrofuran was added dropwise at 30°C, after which the ether was distilled off. The reaction was refluxed for five hours and it was then kept standing at room temperature for about 60 hours. The mixture was then decomposed with aqueous ammonium chloride and filtered. The organic layer was separated off and dried on sodium sulphate and the solvent was distilled off. The residue was purified over a silica gel column with a mixture of chloroform, methanol and 25% ammonia (90:10:2) as the eluent.

α(4-Chlorophenyl)-α-(3,3-dimethylbutyl-1*H*-imidazole-1-ethanol was obtained. Melting point 185°C.

b) A solution of 2.0 g (0.006 mole) of α-(4-chlorophenyl)-α-(3,3-dimethylbutyl)-1*H*-imidazole-1-ethanol in 50 ml of chloroform was saturated with hydrogen chloride gas. The chloroform was distilled off, the residue was dissolved in 30 ml of thionyl chloride and the solution was refluxed with stirring for two hours. The liquid was distilled off and the residue was extracted with ethyl acetate and 4N ammonia. The organic layer was separated off and dried on sodium sulphate and the solvent was distilled off. The residue was taken up in 30 ml of *tert* butanol, after which 2 g of potassium *tert* butoxide were added. The mixture was heated for three hours at 50°C with stirring and the solvent was then distilled off. The residue was extracted with ethyl acetate and water, the organic layer was separated off and dried on sodium sulphate. The solvent was distilled off and the oily residue was dissolved in ethyl acetate. The solution was acidified with an ethereal solution of oxalic acid and the precipitate, consisting of 1-[2-(4-chlorophenyl)-5,5-dimethyl-1-hexenyl]-1*H*-imidazole ethanedioate (1:1) was filtered off, washed with diethyl ether and dried in vacuo at about 60°C. Melting point 136.5°C.

Example 21

a) 100 ml of 15% butyl lithium solution in hexane were slowly added dropwise to a solution of 21.3 g (0.1 mole) of 4-bromo-1-(1,1-dimethylethyl)benzene in 30 ml of diethyl ether. The mixture was stirred for 15 minutes and then 8.0 g (0.05 mole) of 1-(1*H*-imidazole-1-yl)-3,3-dimethyl-2-butanone in 10 ml of diethyl ether were added dropwise. The mixture was stirred for another two hours at room temperature and it was then decomposed with water. The very voluminous precipitate was filtered off and dried in vacuo at about 60°C and then dissolved in ethyl acetate. The solution was acidified with concentrated nitric acid and the precipitated α-(1,1-dimethylethyl)-α-[4-(1,1-dimethylethyl)phenyl]-1*H*-imidazole-1-ethanol nitrate was filtered off and dried in vacuo at about 60°C. Melting point 172°C.

b) 3.1 g (0.008 mole) of α-(1,1-dimethylethyl)-α-[4-(1,1-dimethylethyl)phenyl]-1*H*-imidazole-1-ethanol nitrate were converted into the free base, which was dissolved in 15 ml of hexamethyl phosphortriamide, the solution was refluxed for five minutes and it was then poured into water. The aqueous solution was extracted with diethyl ether and the ethereal solution was washed thoroughly with water. The solution was then dried on sodium sulphate and the solvent was distilled off. The residue was taken up in ethyl acetate and the solution was acidified with concentrated nitric acid. The precipitate was crystallised from a mixture of acetone and diethyl ether. The crystallisation proceeded very slowly. Coarse and fine crystals were formed, which were filtered off and separated by sieving. The fine crystals consisted of the desired 1-[2-[4-(dimethylethyl)phenyl]-3,3-dimethyl-1-butenyl]-1*H*-imidazole nitrate, which was dried in vacuo at about 60°C. Melting point 133°C.

Example 22

a) 100 ml of a 15% solution of butyl lithium in hexane were added dropwise to a solution of 22.5 g (0.1 mole) of 4-bromobenzotrifluoride in 30 ml of diethyl ether. The mixture was stirred for 15 minutes and then a solution of 8 g (0.05 mole) of 1-(1*H*-imidazol-1-yl)-3,3-dimethyl-2-butanone in 30 ml of diethyl ether were added. The mixture was refluxed for two hours. The reaction mixture was then decomposed with a small amount of water and acidified with concentrated hydrochloric acid. The precipitated α-(1,1-dimethyl-ethyl)-α-[4-(trifluoromethyl)phenyl]-1*H*-imidazole-1-ethanol hydrochloride was filtered off and boiled with a little acetone. Melting point 257.1°C.

b) 3.5 g (0.01 mole) of α-(1,1-dimethylethyl)-α-[4-(trifluoromethyl)phenyl]-1*H*-imidazole-1-ethanol hydrochloride were converted into the free base, which was dissolved in 15 ml of hexamethyl phosphortriamide. The mixture was refluxed for 10 minutes at 240°C and it was then cooled and diluted with water and diethyl ether. The ethereal layer was separated off, washed thrice with water and dried on sodium

sulphate. The solvent was distilled off and the oily residue was subjected to chromatography over a silica gel column with a mixture of chloroform, ethyl acetate and 25% ammonia (50:50:2) as the eluent.

1-[3,3-dimethyl-2-[4-(trifluoromethyl)phenyl]-1-butenyl]-1*H*-imidazole was obtained, which was converted into its nitrate with melting point 153.5°C.

Example 23

a) Following the procedure described in Example 22a, but using 3-bromobenzotrifluoride instead of 4-bromobenzotrifluoride, there was obtained α-(1,1-dimethylethyl)-α-[3-(trifluoromethyl)phenyl]-1*H*-imidazole-1-ethanol hydrochloride, of which 3.5 g were converted into the free base.

b) The base obtained as described under a) was converted into 1-[3,3-dimethyl-2-(3-(trifluoromethyl)phenyl-1-butenyl]-1*H*-imidazole nitrate by a similar procedure as described in Example 22b. Melting point 159°C.

Example 24

a) Using a similar procedure as described in Example 22a, α-(4-bromophenyl)-α-(1,1-dimethylethyl)-1*H*-imidazole-1-ethanol hydrochloride was prepared from 1,4-dibromobenzene, butyl lithium and 1-(1*H*-imidazol-1-yl)-3,3-dimethyl-2-butanone. 3.5 g (0.01 mole) of the salt were converted into the free base.

b) The base obtained as described under a) was converted into 1-[2-(4-bromophenyl)-3,3-dimethyl-1-butenyl]-1*H*-imidazole nitrate by a similar procedure as described in Example 22b. Melting point 175.5°C.

Example 25

a) Using a similar procedure as described in Example 22a, α-(1,1-dimethylethyl)-α-(4-ethoxyphenyl)-1*H*-imidazole-1-ethanol hydrochloride was prepared from 1-bromo-4-ethoxybenzene, butyl lithium and 1-(1*H*-imidazol-1-yl)-3,3-dimethyl-2-butanone.

b) A solution of 3.2 g (0.01 mole) of α-(1,1-dimethylethyl)-α-(4-ethoxyphenyl)-1*H*-imidazole-1-ethanol hydrochloride in 10 ml of hexamethylphosphortriamide was refluxed for 5 minutes. The mixture was cooled and then dilute ammonia and diethyl ether were added. The ethereal layer was separated off, washed thrice with water and dried on sodium sulphate. The solvent was distilled off and the basic residue was converted into 1-[2-(4-ethoxyphenyl)-3,3-dimethyl-1-butenyl]-1*H*-imidazol hydrochloride with melting point 222.5°C.

Example 26

a) Using a similar procedure as described in Example 22a, α-(1,1-dimethyl)-α-(4-fluorophenyl)-1*H*-imidazole-1-ethanol hydrochloride was prepared from 1-bromo-4-fluorobenzene, butyl lithium and 1-1*H*-imidazol-1-yl-3,3-dimethyl-2-butanone.

b) The compound obtained as described under a) was converted into 1-[2-(4-fluorophenyl)-3,3-dimethyl-1-butenyl]-1*H*-imidazole hydrochloride by a similar procedure as described in Example 25b. Melting point 233°C.

Example 27

a) Following the procedure described in example 5a, but using (4-chlorophenyl)cyclohexylmethanone instead of 1-(3,4-dichlorophenyl)-2,2-dimethyl-1-propanone, there was obtained 2-(4-chlorophenyl)-2-cyclohexyloxirane.

b) A mixture of 20 g (0.0845 mole) of 2-(4-chlorophenyl)-2-cyclohexyloxirane and 34 g (0.5 mole) of 1*H*-imidazole was heated for two hours with stirring at 160°C. The mixture was cooled and it was then extracted with chloroform and water. The organic layer was washed several times with water and dried on sodium sulphate. The solvent was distilled off and the residue was boiled with acetone. The mixture was cooled and then diethyl ether was added and the solid was filtered off and dried in vacuo at about 60°C.

α-(4-Chlorophenyl)-α-cyclohexyl-1*H*-imidazole-1-ethanol was obtained. Melting point >200°C.

c) A solution of 11.5 g (0.0377 mole) of α-(4-chlorophenyl)-α-cyclohexyl-1*H*-imidazole-1-ethanol in 50 ml of hexamethylphosphortriamide was heated for five minutes at 235°C and subsequently poured into water. The mixture was extracted with diethyl ether and the extract was dried on sodium sulphate. The ether was distilled off and the residue was taken up in acetone and acidified with an ethereal hydrogen chloride solution. The precipitated 1-[2-(4-chlorophenyl)-2-cyclohexylethenyl]-1*H*-imidazole hydrochloride was filtered off, crystallised from a mixture of 2-propanol and diethyl ether and dried in vacuo at about 60°C.

Melting point 212°C.

Example 28

a) A Grignard solution, prepared from 31 g (0.2 mole) of 1-bromohexane and 4.8 g (0.2 at) of magnesium in 120 ml of diethyl ether was added very slowly at 0—5°C to a suspension of 44.7 g (0.2 mole) of 2,2′,4′-trichloroacetophenone in 200 ml of diethyl ether. The mixture was stirred for one hour at 0°C, the cooling means were then removed and the reaction mixture was kept standing overnight.

It was then decomposed with an aqueous ammonium chloride solution. The organic layer was separated off and dried on sodium sulphate. The solvent was distilled off and the residue was distilled. 2,4-

Dichloro-α-(chloromethyl)-α-hexylbenzenemethanol was obtained. Boiling point 112—150°C/0.4 mm Hg.

b) A mixture of 8.0 g (0.026 mole) of crude 2,4-dichloro-α-(chloromethyl)-α-hexylbenzenemethanol and 10 g (0.147 mole) of 1*H*-imidazole were heated for two hours at 120°C with stirring. The reaction mixture was cooled and it was then extracted with water and chloroform. The organic layer was separated off and dried on sodium sulphate. The solvent was distilled off and the residue was purified over a silica gel column with a mixture of chloroform, ethyl acetate and 25% ammonia (50:50:1) as the eluent. The solid obtained after evaporation of the solvent was dried in vacuo at 50°C.

α-(2,4-Dichlorophenyl)-α-hexyl-1*H*-imidazole-1-ethanol was obtained.

c) A solution of 3.2 g (0.0094 mole) of α-(2,4-dichlorophenyl)-α-hexyl-1*H*-imidazole-1-ethanol in 15 ml of hexamethylphosphortriamide was refluxed for two hours and then poured into water. The mixture was extracted with ethyl acetate and the extract was dried on sodium sulphate. The solvent was distilled off and the residue consisting of impure 1-[2-(2,4-dichlorophenyl)-1-octenyl]-1*H*-imidazole was converted into the oxalate. The base was liberated again, purified over a silica gel column with a mixture of chloroform, ethyl acetate and 25% ammonia (50:50:1) as the eluent and converted into the oxalate (1:1) (ethanedioate). Melting point 119°C.

### Example 29

a) α-(1,1-dimethylethyl)-α-(3-chlorophenyl)-1*H*-imidazole-1-ethanol hydrochloride was prepared from 1-bromo-3-chlorobenzene, butyl lithium and 1-(1*H*-imidazol-1-yl)-3,3-dimethyl-2-butanone in a similar manner as described in Example 22a.

b) 3.1 of the salt obtained as described under a) were converted into the free base, which was dissolved in 10 ml of hexamethyl phosphortriamide. The mixture was refluxed for 10 minutes at 240°C and it was then cooled and diluted with water and diethyl ether. The ethereal layer was separated off, washed three times with water and dried on sodium sulphate. The solvent was distilled off and the residue consisting of 1-[2-(3-chlorophenyl)-3,3-dimethyl-1-butenyl]-1*H*-imidazole, was converted into the hydrochloride.

Melting point 225°C.

### Example 30

a) 65 ml of a 15% butyl lithium solution in hexane were added dropwise at a temperature between 0 and −10°C to a solution of 14 ml (0.1 mole) of diisopropylamine in 150 ml of anhydrous tetrahydrofuran. The mixture was stirred for one hour at −10°C and then a solution of 10.7 g (0.1 mole) of imidazole-1-acetonitrile in 75 ml of tetrahydrofuran and a solution of 19.8 g (0.1 mole) of 1-(4-chlorophenyl)-2,2-dimethyl-1-propanone (p-chloropivalophenon, prepared as described by G. Tsatsas and G. Cotakis, Bull.Soc.Chim. France, *1970*, 3609—3616) in 100 ml of tetrahydrofuran were succesively added dropwise at −60°C. The reaction mixture was allowed to attain room temperature and it was then refluxed for five hours. The mixture was cooled and poured into a saturated sodium chloride solution. The organic layer was separated off and the solvent was distilled off. The residue was dissolved in diethyl ether and the solution was washed with water, dried on magnesium sulphate and acidified with an ethereal hydrogen chloride solution. The oily precipitate was separated off, made alkaline with ammonia and extracted with diethyl ether. The solvent was distilled off and the residue was purified over a silica gel column with a mixture of chloroform, ethyl acetate and 25% ammonia (50:50:1) as the eluent.

α-[1-(4-Chlorophenyl)-2,2-dimethylpropylidene]-1*H*-imidazole-1-acetonitrile was obtained. Melting point 97°C.

b) A mixture of 1 g of α-[1-(4-chlorophenyl)-2,2-dimethylpropylidene]-1*H*-imidazole-1-acetonitrile and 0.5 g of sodium hydroxide in 15 ml of ethylene glycol was refluxed for three hours. The reaction mixture was neutralised with a solution of hydrogen chloride in ethanol and the ethylene glycol was then distilled off in vacuo on a water bath. The residue was taken up in chloroform, the solution was washed with water and the solvent was distilled off. The crude acid thus obtained was heated for 5 minutes at 200—230°C, which caused evolution of carbon dioxide. The decarboxylated product was purified by column chromatography (silica gel) with a mixture of chloroform, ethyl acetate and 25% ammonia (50:50:1) as the eluent. A fraction was isolated with the same rf-value as 1-[2-(4-chlorophenyl)-3,3-dimethyl-1-butenyl]-1*H*-imidazole (Z-isomer) obtained in Example 6b. From the NMR spectrum it could be concluded that the product was a mixture of the E- and Z-isomers in the ratio 3:1. On prolonged heating at 200°C the E isomer is converted into the more stable Z-isomer.

### Example 31

a) A mixture of 2.5 g of α-[1-(4-chlorophenyl)-2,2-dimethylpropylidene]-1*H*-imidazole-1-acetonitrile (see Example 30a), 60 ml of ethanol and 60 ml of 2N sodium hydroxide solution was refluxed for 8 hours. The reaction mixture was cooled and a part of the liquid was evaporated. Diethyl ether and water were then added and the solid formed was filtered off and dried in vacuo at about 60°C.

α-[1-(4-Chlorophenyl)-2,2-dimethyl-1-propylidene]-1*H*-imidazol-1-acetamide was obtained. Melting point 250°C.

b) A mixture of 1 g of α-[1-(4-Chlorophenyl)-2,2-dimethyl-1-propylidene]-1*H*-imidazol-1-acetamide, 15 ml of ethylene glycol and 0.5 g of sodium hydroxide was refluxed with stirring for three hours. The mixture was cooled and neutralised with an ethanolic hydrogen chloride solution. The solvent was then

distilled off in vacuo (oil pump) on a boiling water bath. The residue (carboxylic acid) was heated for 10 minutes at 220°C. According to thin layer chromatography (colour with potassium iodine platinate) the resulting oil contained the E-isomer of 1-[2-(4-chlorophenyl)-3,3-dimethyl-1-butenyl]-1*H*-imidazole.

Example 32

a) 2-(4-Chlorophenyl)-2-ethyloxirane was prepared from 1-(4-chlorophenyl)-1-propanone and dimethyloxosulfonium methylide by a similar procedure as described in Example 5a.

b) α-(4-Chlorophenyl)-α-ethyl-1*H*-imidazol-1-ethanol was prepared from 2-(4-chlorophenyl)-2-ethyloxirane and 1*H*-imidazole by a similar procedure as described in Example 5b.

c) 13.8 g (0.055 mole) of the product obtained as described under b was dissolved in 300 ml of chloroform and the solution was saturated with hydrogen chloride gas. The solvent was evaporated, the residue was taken up in 100 ml of thionyl chloride and the clear solution was refluxed for two hours with stirring.

The liquid was evaporated and the residue was boiled a few times with a mixture of acetone and diethyl ether and filtered. The filtrate was further processed as described under d. The undissolved solid (mainly consisting of 1-[2-chloro-2-(4-chlorophenyl)butyl]-1*H*-imidazole) was mixed with 10 g of potassium *tert* butoxide and 50 ml of *tert* butanol and the mixture was heated for two hours at 50°C with stirring. The solvent was evaporated and the residue was extracted with diethyl ether and water. The organic phase was separated off, dried on sodium sulphate and the ether was evaporated. The residue was dissolved in acetone and the solution was acidified with an ethereal hydrogen chloride solution. The precipitate formed was filtered off, washed with acetone and diethyl ether and dried in vacuo at about 60°C. The spectra showed that the product was a mixture of the two isomers of 1-[2-(4-chlorophenyl)-1-butenyl]-1*H*-imidazole hydrochloride. Melting point 133°C.

d) The filtrate obtained in step c was concentrated and 50 ml of *tert* butanol and 10 g of potassium *tert* butoxide were added to the residue. The mixture was heated for two hours at 50°C with stirring, after which the solvent was evaporated. The residue was extracted with a mixture of diethyl ether and water. The organic layer was separated off and dried on sodium sulphate and the solvent was evaporated. The residue was dissolved in acetone and the solution was acidified with a solution of hydrogen chloride in diethyl ether. The precipitate was filtered off and crystallised from a mixture of 2-propanol and diethyl ether. One of the isomers of 1-[2-(4-chlorophenyl)-1-butenyl]-1*H*-imidazole hydrochloride was obtained. Melting point 158°C.

Example 33

a) α-(1,1-dimethylethyl)-α-(2-fluorophenyl)-1*H*-imidazol-1-ethanol was prepared at −50°C from 1-bromo-2-fluorobenzene, butyl lithium and 1-(1*H*-imidazol-1-yl)-3,3-dimethyl-2-butanone by a similar procedure as described in Example 22a. The base was purified by column chromatography (silica gel; chloroform/ethyl acetate/ammonia 50:50:1) and converted into the hydrochloride, melting point 238.5°C.

b) A solution of 1.3 g (0.004 mole) of α-(1,1-dimethylethyl)-α-(2-fluorophenyl)-1*H*-imidazole-1-ethanol hydrochloride in 10 ml of hexamethyl phosphortriamide was refluxed for five minutes. After cooling, dilute ammonia and diethyl ether were added to the reaction mixture. The ethereal layer was separated off, washed thrice with water and dried on sodium sulphate and the ether was evaporated. The oil obtained was converted into the hydrochloride. The salt was boiled with a little acetone, filtered off and dried in vacuo at about 60°C.

1-[2-(2-fluorophenyl)-3,3-dimethyl-1-butenyl]-1*H*-imidazole hydrochloride was obtained. Melting point 238.5°C.

Example 34

a) Using a similar procedure as described in Example 5a, 2-(4-chlorophenyl)-2-(1-methylpropyl)oxirane was prepared from 1-(4-chlorophenyl)-2-methyl-1-butanone and dimethyloxosulfonium methylide.

b) Using a similar procedure as described in Example 5b, α-(4-chlorophenyl)-α-(1-methylpropyl)-1*H*-imidazole-1-ethanol hydrochloride was prepared from 2-(4-chlorophenyl)-2-(1-methylpropyl)oxirane and 1*H*-imidazole. Melting point 170°C.

c) 5 ml of thionyl chloride were added dropwise at 0°C with thorough stirring to a mixture of 10 g of α-(4-chlorophenyl)-α-(1-methylpropyl)-1*H*-imidazole-1-ethanol hydrochloride and 70 ml of anhydrous pyridine. The mixture was stirred for one hour at 0°C and it was then poured into ice water. The mixture obtained was then extracted with diethyl ether. The organic layer was washed five times with water and dried on sodium sulphate and the solvent was evaporated. The residue was purified by column chromatography (silica gel; chloroform/ethyl acetate/25% ammonia 50:50:1). Two fractions were obtained, of which the first one yielded the desired 1-[2-(4-chlorophenyl)-3-methyl-1-pentenyl]-1*H*-imidazole. The base was converted into the hydrochloride, which was crystallised from a mixture of acetone and diethyl ether. Melting point 170°C. In the preparation of a larger quantity, the E and Z isomers crystallised separately, melting points respectively 199.5°C and 201°C.

Example 35

a) (2,4-Dichlorophenyl)-2-methyloxirane was prepared from 1-(2,4-dichlorophenyl)-1-ethanone and dimethyloxosulfonium methylide by a similar procedure as described in Example 5a.

b) α-(2,4-Dichlorophenyl)-α-methyl-1*H*-imidazole-1-ethanol was prepared from (2,4-dichlorophenyl)-2-methyloxirane and 1*H*-imidazole by a similar procedure as described in Example 5b.

c) 13 ml of thionyl chloride were added dropwise at 0°C to a solution of 16.7 g (0.06 mole) of α-(2,4-dichlorophenyl)-α-methyl-1*H*-imidazole-1-ethanol in 92.5 ml of pyridine. Stirring was continued for one hour at 0°C and two hours at room temperature and the suspension obtained was then poured into water. The mixture was then extracted with ethyl acetate, the extract was washed several times with water and dried and the solvent was evaporated. The residue was taken up in acetone and the resulting solution was acidified with an ethereal hydrogen chloride solution. The precipitate was filtered off and crystallised from a mixture of ethanol and diethyl ether.

1-[2-Chloro-2-(2,4-dichlorophenyl)propyl]-1*H*-imidazole hydrochloride was obtained. Melting point 177°C.

d) A mixture of 14 g (0.043 mole) of 1-[2-chloro-2-(2,4-dichlorophenyl)propyl]-1*H*-imidazol hydrochloride, 10 g of potassium *tert* butoxide and 60 ml of *tert* butanol was heated for 4 hours at 70°C with stirring. The solvent was then evaporated and the residue was extracted with water and ethyl acetate. The organic layer was separated off and dried on sodium sulphate and the solvent was evaporated. The residue was taken up in 2-propanol and acidified with an ethereal hydrogen chloride solution. The precipitate was filtered off and crystallised twice from a mixture of 2-propanol and diethyl ether after decolorization with active charcoal. Finally, the solid product was boiled with acetone, filtered and dried in vacuo at about 60°C.

1-[2-(2,4-Dichlorophenyl)-1-propenyl]-1*H*-imidazole hydrochloride was obtained. Melting point 160.5°C.

Example 36

a) Using a similar procedure as described in Example 14a, α-(2,5-dichlorophenyl)-α-(1,1-dimethylethyl)-1*H*-imidazole-1-ethanol was prepared from 1-(2,5-dichlorophenyl)-2-(1*H*-imidazol-1-yl)ethanone and *tert* butyl magnesium chloride.

The crude product was purified on a silica gel column with dichloromethane/methanol as the eluent. Melting point 158°C.

b) A mixture of 2 g of α-(2,5-dichlorophenyl)-α-(1,1-dimethylethyl)-1*H*-imidazole-1-ethanol and 5 ml of hexamethyl phosphortriamide was heated at 235—240°C for five minutes. The reaction mixture was then cooled and poured into water and the resulting mixture was extracted with diethyl ether. The solvent was evaporated and the residue was dissolved in 2-propanol. The solution thus obtained was acidified with a solution of hydrogen chloride gas in 2-propanol. The substance first crystallising was the hydrochloride of the starting compound. After filtration and addition of diethyl ether, 1-[2-(2,5-dichlorophenyl)-3,3-dimethyl-1-butenyl]-1*H*-imidazole hydrochloride crystallised. The product was filtered off and dried in vacuo at about 60°C. Melting point 234°C.

Example 37

a) Using a similar procedure as described in Example 5a, 2-(2-chlorophenyl)-2-ethyloxirane was prepared from 1-(2-chlorophenyl)-1-propanone and dimethyloxosulfonium methylide.

b) Using a similar procedure as described in Example 5b, α-(2-chlorophenyl)-α-ethyl-1*H*-imidazol-1-ethanol was prepared from 2-(2-chlorophenyl)-2-ethyloxirane and 1*H*-imidazole. The base was dissolved in 2-propanol and converted into the hydrochloride by addition of a solution of hydrogen chloride gas in 2-propanol. On addition of diethyl ether and petroleum ether (boiling range 40—60°C) the salt precipitated. It was filtered off and dried in vacuo at about 60°C. Melting point 181.5°C.

c) 11 ml of thionyl chloride were added dropwise at 0°C to a solution of 14 g of α-(2-chlorophenyl)-α-ethyl-1*H*-imidazole-1-ethanol hydrochloride in 70 ml of pyridine. The mixture was stirred for 30 minutes at 0°C and for 1.5 hours at 20°C and it was then poured into a mixture of ice and water. The resulting mixture was extracted with ethyl acetate, the extract was dried on sodium sulphate and the solvent was evaporated. The oily residue was purified by column chromatography (silica gel; chloroform/ethyl acetate/25% ammonia 50:50:1) and the base obtained was converted into its hydrochloride.

1-[2-(2-chlorophenyl)-1-butenyl]-1*H*-imidazole hydrochloride was obtained. Melting point 135°C.

Example 38

a) α-(2,6-Difluorophenyl)-α-(1,1-dimethylethyl)-1*H*-imidazole-1-ethanol hydrochloride was prepared at −50°C in tetrahydrofuran from 1,3-difluorobenzene, butyl lithium and 1-(1*H*-imidazol-1-yl)-3,3-dimethyl-2-butanone by a similar procedure as described in Example 22a. Melting point 237.5°C.

b) A solution of 5.0 g of α-(2,6-difluorophenyl)-α-(1,1-dimethylethyl)-1*H*-imidazole-1-ethanol hydrochloride in 30 ml of hexamethyl phosphortriamide was refluxed for ten minutes. After cooling, dilute ammonia and diethyl ether were added. The ethereal layer was separated off, washed thrice with water and dried on sodium sulphate and the ether was evaporated. The residue was chromatographed over a silica gel column with chloroform/ethyl acetate/25% ammonia 50:50:1 as the eluent. The base obtained was converted into its hydrochloride.

1-[2-(2,6-Difluorophenyl)-3,3-dimethyl-1-butenyl]-1*H*-imidazole hydrochloride was obtained. Melting point 237.5°C.

Example 39

a) Using a similar procedure as described in Example 5a, 2-(2-chlorophenyl)-2-propyloxirane was prepared from 1-(2-chlorophenyl)-1-butanone and dimethyloxosulfonium methylide.

b) α-(2-Chlorophenyl)-α-propyl-1*H*-imidazole-1-ethanol was prepared from 2-(2-chlorophenyl)-2-propyloxirane and 1*H*-imidazole by the method described in Example 37b.

c) α-(2-Chlorophenyl)-α-propyl-1*H*-imidazole-1-ethanol was converted into 1-[2-(2-chlorophenyl)-1-pentenyl]-1*H*-imidazole hydrochloride by the method described in Example 37c. Melting point 184°C.

Example 40

a) 2,4-Dichlorobenzaldehyde was reacted with cyclopropyl lithium to give α-cyclopropyl-α-(2,4-dichlorophenyl)methanol. 108.5 g (0.5 mole) of this compound in 150 ml of glacial acetic acid was oxidized into the corresponding ketone by 60 g (0.2 mole) of potassium bichromate in 180 ml of water and 45 ml of concentrated sulfuric acid.

b) The ketone obtained was reacted with dimethyloxosulfonium methylide in the manner described in Example 5a, to give 2-cyclopropyl-2-(2,4-dichlorophenyl)oxirane. Reaction of this compound with 1*H*-imidazole, in the manner described in Example 5b, yielded α-cyclopropyl-α-(2,4-dichlorophenyl)-1*H*-imidazole-1-ethanol hydrochloride. Melting point 195.5°C.

c) A solution of 4.3 g (0.013 mole) of α-cyclopropyl-α-(2,4-dichlorophenyl)-1*H*-imidazole-1-ethanol hydrochloride in 40 ml of hexamethyl phosphortriamide was refluxed for 5 minutes. The reaction mixture was cooled and 4N ammonia and diethyl ether were added. The ethereal layer was separated off, washed thrice with water and dried on sodium sulphate. The ether was evaporated and the residue was purified by column chromatography (silica gel, chloroform/ethyl acetate/25% ammonia 50:50:1). The base obtained was converted into its hydrochloride, which was dried in vacuo at about 60°C. There was obtained 1-[2-cyclopropyl-2-(2,4-dichlorophenyl)ethenyl]-1*H*-imidazole hydrochloride. Melting point 195.5°C.

Example 41

a) Using a similar procedure as described in Example 5a, 2-(2,4-dichlorophenyl)-2-(1-methylpropyl)oxirane was prepared from 1-(2,4-dichlorophenyl)-2-methyl-1-butanone and dimethyloxosulfonium methylide.

b) Using a similar procedure as described in Example 5b, α-(2,4-dichlorophenyl)-α-(1-methylpropyl)-1*H*-imidazol-1-ethanol was prepared from 2-(2,4-dichlorophenyl)-2-(1-methylpropyl)oxirane and 1*H*-imidazole.

c) A mixture of 6.6 g of α-(2,4-dichlorophenyl)-α-(1-methylpropyl)-1*H*-imidazole-1-ethanol and 100 ml of thionyl chloride was refluxed for four hours with stirring. The reaction mixture was then cooled and the liquid was evaporated. The residue was crystallised first from a mixture of 2-propanol and diethyl ether and then from a mixture of acetone and diethyl ether.

1-[2-(2,4-Dichlorophenyl)-3-methyl-1-pentenyl]-1*H*-imidazole hydrochloride was obtained. Melting point 207°C.

Example 42

a) 46 g (2 mole) of sodium were dissolved at −76°C in 1.75 l of anhydrous liquid ammonia. Following 40 minutes stirring at −76°C, 262 g (1 mole) of triphenylphosphine were added at −60 to −70°C in the course of 15 minutes. The mixture was stirred for 2.5 hours and then 50 g (0.93 mole) of ammonium chloride (which had been dried at 110°C) was added. Stirring was continued for one hour and the reaction mixture was then added to 1.2 l of dichloromethane of −60°C in 30 minutes by means of nitrogen pressure. The last remainders were washed over with 200 ml of dichloromethane. Stirring was continued for 30 minutes without cooling, whereafter 18 ml of water were added, which raised the temperature to −25°C, followed by 60 ml of 30% hydrogen peroxide. After stirring overnight without cooling, the mixture was extracted two times with 100 ml of a 2 M sodium sulphite solution and once with 100 ml of a 20% ammonium chloride solution. The organic layer was dried, filtered and concentrated. The residue was crystallised from a mixture of 200 ml of ethyl acetate and 75 ml of dichloromethane, giving 57% (chloromethyl)diphenylphosphine oxide, melting point 133—135°C. Another 15% could be obtained by chromatography of the mother liquor (silica gel, ethyl acetate/methanol 95:5 as the eluent).

b) 23.3 g (0.342 mole) of 1*H*-imidazole were added in small portions under a nitrogen atmosphere to a suspension of 8.25 g (0.343 mole) of sodium hydride in 460 ml of anhydrous dimethylformamide with thorough stirring, on which the temperature rose to 50°C. The mixture was stirred for 10 minutes and then 83.5 g (0.333 mole) of (chloromethyl)diphenylphosphine oxide were added, which gave a temperature rise to 41°C. The reaction mixture was stirred at 80°C for six hours and then it was cooled and the precipitate was filtered off. The filtrate was concentrated at 50°C/1 mm Hg. The residue was dissolved in 350 ml of boiling dichloromethane. After adding 250 ml of boiling ethyl acetate the product began to crystallise. The mixture was allowed to cool, the precipitate was filtered off, washed with cold ethyl acetate and dried, affording 71% [(1*H*-imidazol-1-yl)methyl]diphenylphosphine oxide. Another 4% could be obtained by

crystallising the mother liquor from ethyl acetate. Melting point 175°C.

c) 50 ml of a 1.5 M butyl lithium solution in hexane (0.075 mole) were added dropwise at −30°C to a suspension of 14.1 g (0.05 mole) of [(1*H*-imidazol-1-yl)methyl]diphenylphosphine oxide in 500 ml of anhydrous tetrahydrofuran. The mixture was stirred at −30°C for 10 minutes and then a solution of 11 g (0.056 mole) of 1-(4-chlorophenyl)-2,2-dimethyl-1-propanone (= 4-chloropivalophenone) in 25 ml of tetrahydrofuran was added dropwise. After 20 minutes stirring at −30°C, the reaction mixture was poured into 500 ml of a 20% ammonium chloride solution and stirred vigorously. The aqueous mixture was extracted thrice with toluene, the extract was dried and the solvent was distilled off. The residue was taken up in 100 ml of diethyl ether and cooled to −10°C and then 3.5 ml of 65% nitric acid were added dropwise. The precipitate was filtered off, dried in vacuo and crystallised from 2-propanol.

1-[2-(4-Chlorophenyl)-3,3-dimethyl-1-butenyl]-1*H*-imidazole nitrate was obtained. Melting point 160°C.

By a similar procedure, the compound 1-[2-(2,4-dichlorophenyl)-3,3-dimethyl-1-butenyl]-1*H*-imidazole nitrate was obtained. Melting point 189.5°C.

## Example 43

a) 320 ml of a 0.6N ethereal solution of allyl magnesium bromide were added dropwise to a refluxing solution of 22.0 g (0.1 mole) of 1-(4-chlorophenyl)-2-(1*H*-imidazol-1-yl)-1-ethanone (E. Godefroi et al. J.Med.Chem. *12*, 784 (1969)) in 250 ml of anhydrous tetrahydrofuran. During the addition the ether was distilled off. The mixture was refluxed for one hour and it was then decomposed with an aqueous ammonium chloride solution. The organic layer was separated off and dried on sodium sulphate and the solvent was evaporated. Ethyl acetate was added, which made the residue crystallise. The solid was filtered off, washed with ethyl acetate and diethyl ether and dried in vacuo at about 60°C.

α-(4-Chlorophenyl)-α-(2-propenyl)-1*H*-imidazole-1-ethanol was obtained.

b) A solution of 5.6 g (0.021 mole) of α-(4-chlorophenyl)-α-(2-propenyl)-1*H*-imidazole-1-ethanol in 100 ml of chloroform was saturated with hydrogen chloride gas. The solvent was evaporated and the residue was dissolved in 50 ml of thionyl chloride. The solution was refluxed for three hours and the solvent was subsequently evaporated. The residue was shaken with 2N ammonia and ethyl acetate. The organic layer was separated off and dried on sodium sulphate and the solvent was evaporated. To the residue 50 ml of *tert* butanol and 6 g of potassium *tert* butoxide were added, after which the mixture was stirred at 60°C for three hours. The liquid was evaporated and the residue was extracted with chloroform and water. The organic layer was separated off and dried on sodium sulphate and the solvent was evaporated. The residue was purified over a silica gel column with chloroform/ethyl acetate/25% ammonia (50:50:1) as the eluent. The appropriate fractions were combined and the solvent was evaporated. The oily residue was dissolved in ethyl acetate and the solution was acidified with a 20% solution of hydrogen chloride gas in 2-propanol. The precipitated salt was filtered off, washed with diethyl ether and dried in vacuo at about 60°C.

1-[2-(4-Chlorophenyl)-1,3-pentadienyl]-1*H*-imidazole hydrochloride (mixture of isomers) was obtained. Melting point 140°C (with decomposition).

Remark

During the treatment with potassium butoxide a shift of the propenyl double bond takes place, so that a conjugated system is formed.

The compounds of the invention were tested in vitro and in vivo. The in vitro activities were determined by means of the usual agar dilution test. The results after two and five days were expressed as MIC 50 and MIC 90, i.e. the minimum concentrations giving complete inhibition of 50% or 90% of the strains tested in micrograms per litre and as Potency Ratio (P.R.), i.e. the ratio between the activity of the test compound and the activity of miconazol (= 1).

The following methods were used for in vivo examination.

A. Testing of compounds against systemic infection with Candida albicans in mice.

Female Swiss SPF mice, weighing 10—21 g, ten animals per group, were challenged intravenously with 0.5 ml Candida albicans strain A 7 suspension containing sufficient yeast cells to kill 50% of the untreated animals within 5 days. The treatment consisted of three doses daily administered for 3 days either orally or intraperitoneally. Infected, non treated mice served as controls for mortality. The number of mice surviving the challenge was recorded daily and the ratio between the "median survival duration (days)" of the treated and untreated mice was determined, the survival duration of the control animals being graded as 1 (IMSD = Index Median Survival Duration).

B. Testing of compounds against vaginitis candidamycetica in mice (experimentally induced infection). ("vaginal reduction")

Female Swiss SPF mice, weighing 19—21 g, fifteen animals per group, following an intramuscular premedication by Dimenformon prolongatum, were inoculated intravaginally with $\pm 5.10^5$ CFU of Candida albicans strain A 7, harvested from a culture on Mycological Agar and suspended in physiological saline.

The treatment commenced 72 hours after infection when the candidiasis had reached its peak. A four day local treatment period was employed, consisting of two applications daily of 0.02 ml of antimycotics in

Plastibase intravaginally. Alternatively, the test compound was administered orally in three doses daily for three days.

On the seventh day after infection the mice were killed, the vagina was excised and homogenized in saline and cultivated on Mycological Agar. The yeast cells were then counted and expressed as CFU/vagina. Results wee analysed statistically using the T-test. The results were compared with those obtained with clotrimazol, the action of which was considered as 100%.

C. Testing of compounds against experimental dermatophytic infection. (Trichophyton mentagrophytes)

Out-bred blanco TNO female guinea pigs, five animals per group, weighing ±350 g, were prepared by depilation high in the middle of the back over an area 2.5 cm in diameter.

Inoculum was prepared from a culture of Trichophyton mentagrophytes strain R177 on Mycological Agar. The harvested mycelial mat was diluted in a 20% dextrose solution in water and the suspension was smeared over the depilated area.

Local treatment with 2% concentration of the antimycotic in Plastibase started 72 hours after infection and concurrently the application of vehiculum (Plastibase) to the infected animals was initiated. The application was continued for 10 days and consisted of two applications daily of a measured quantity of ointment to the infected area.

Scoring of lesions and the final check was made on the 14th day after the infection was initiated. The lesions were graded as to the following parameters:

1=slight whitish scaling, with erythema
2=slight whitish or light yellow scaling with solitary haemorrhagias
3=thick whitish-yellow crust or scab
4=haemorrhagic ulcer or giant haemorrhagic thick scutula (crust).

The final results were recorded as the average of lesion intensities (A.L.I.) for each experimental group (treated with either test compound or reference compound and untreated control). Untreated animals showed an A.L.I. of 4; the reference compound gave an A.L.I. of 0.5—1.5.

Some typical test results are shown in Tables II and III. The structure of the test compounds is given in Table I.

TABLE I

| Compound | $(R_1)_n$ | $R_2$ | $R_3,R_4$ | Salt |
|---|---|---|---|---|
| I | 2,4-$Cl_2$ | n-$C_3H_7$ | H | HCl |
| II | 4-Cl | 2-$C_4H_9$ | H | HCl |
| III | 2,4-$Cl_2$ | 2-$C_4H_9$ | H | HCl |
| IV | 2,4-$Cl_2$ | *tert*-$C_4H_9$ | H | $HNO_3$ |
| V | 4-Cl | cyclohexyl | H | HCl |
| VI | 2,4-$Cl_2$ | cyclohexyl | H | HCl |

TABLE II

In vitro activities

| Compound | | Candida albicans | | Trichophyton spp. | |
|---|---|---|---|---|---|
| | | MIC 50 | MIC 90 | MIC 50 | MIC 90 |
| I | 2 d | 3 | 8 | | |
| | P.R. | 1.0 | 1.2 | | |
| | 5 d | 4 | 9 | 0.02 | 0.15 |
| | P.R. | 1.0 | 0.7 | 35.3 | 13.9 |
| II | 2 d | 2 | 5 | | |
| | P.R. | 1.7 | 1.7 | | |
| | 5 d | 4 | 6 | 0.26 | 1.20 |
| | P.R. | 1.2 | 1.9 | 2.8 | 2.1 |
| III | 2 d | 5 | 10 | | |
| | P.R. | 0.7 | 0.9 | | |
| | 5 d | 6 | 11 | 0.21 | 1.62 |
| | P.R. | 0.7 | 1.2 | 1.7 | 0.8 |
| IV | 2 d | 3 | 5 | | |
| | P.R. | 0.7 | 0.9 | | |
| | 5 d | 5 | 11 | 0.09 | 1.62 |
| | P.R. | 0.5 | 0.5 | 15.1 | 4.1 |
| V | 2 d | 3 | 8 | | |
| | P.R. | 1.3 | 1.0 | | |
| | 5 d | 5 | 10 | 0.96 | 4.5 |
| | P.R. | 1.1 | 1.1 | 0.8 | 0.6 |
| VI | 2 d | 4 | 8 | | |
| | P.R. | 0.9 | 0.7 | | |
| | 5 d | 5 | 11 | 0.46 | 2.37 |
| | P.R. | 0.8 | 0.6 | 1.9 | 1.0 |

TABLE III

In vivo activities

| | Candida alb. meth. A | meth. B | Trich. ment. meth. C |
|---|---|---|---|
| Compound | IMSD i.p. p.o. | vag. red. % | A.L.I. |
| I | 1.4 | 35 | 0 |
| | 3.0 | | |
| II | 3.8 | 98 | 0 |
| | 4.6 | | |
| III | 3.5 | 136 | 0 |
| | 4.1 | | |
| IV | 5.1 | 59 | 0 |
| | 3.1 | 24 (p.o.) | |
| V | 3.5 | | |
| | 4.1 | 150 (p.o.) | |
| VI | 3.7 | 97 | 0.4 |
| | 3.9 | 52 (p.o.) | |

The invention includes within its scope pharmaceutical compositions comprising one or more ethenylimidazole derivatives of formula I. The pharmaceutical compositions may be in a form suited for oral, topical, intraperitoneal, parenteral or rectal administration and they may be prepared by methods customarily employed in pharmaceutical practice. The oral administration is of utmost importance as the compounds of the invention are relatively well resorbed. Tablets or capsules may, for instance, be used for oral application. Preparations for topical application may be in the form of powders, creams, ointments, or sprays. Other pharmaceutical dosage forms may also be used for administering the compounds of the invention.

In addition to the active ingredient, the pharmaceutical compositions may contain a suitable carrier, for instance a dermatological ointment base such as polyethylene 400 or plastibase, and other auxiliary substances used in pharmacy. The amount of active substance in, for instance, an ointment is preferably between 0.5 and 10%, 2% being particularly preferred.

**Claims for the Contracting States: BE CH DE FR GB IT LI LU NL SE**

1. Ethenylimidazole derivatives of the formula

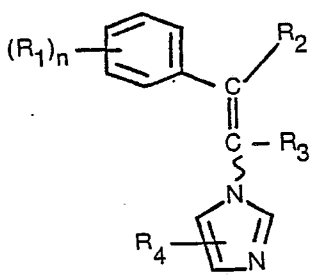

I

in which n is 0, 1 or 2, $R_1$ represents a halogen atom, a trifluoromethylgroup or an alkyl or alkoxy group with at most 6 carbon atoms, the two substituents being the same or different when n is 2, $R_2$ represents an

alkyl group with 1 to 6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or a alkenyl group with 2 to 4 carbon atoms and $R_4$ represents a hydrogen atom or an alkyl group with 1 to 6 carbon atoms, and their acid addition salts.

2. Ethenylimidazole derivatives according to claim 1, in which $R_1$ is chlorine, n is 1 or 2, $R_2$ is an alkyl group with 2—4 carbon atoms or cyclohexyl and $R_3$ and $R_4$ are hydrogen atoms.

3. Ethenylimidazole derivatives according to claim 1, in which $(R_1)_n$ is 4-chloro or 2,4-dichloro.

4. Ethenylimidazole derivatives according to claim 2 or 3, in which $R_2$ is n-propyl, *sec*-butyl, *tert*-butyl or cyclohexyl.

5. 1 - [2 - (2,4 - dichlorophenyl) - 3,3 - dimethyl - 1 - butenyl] - 1*H* - imidazole and its acid addition salts.

6. 1 - [2 - cyclohexyl - 2 - (2,4 - dichlorophenyl)ethenyl] - 1*H* - imidazole and its acid addition salts.

7. 1 - [2 - (4 - chlorophenyl) - 2 - cyclohexylethenyl] - 1*H* - imidazole and its acid addition salts.

8. 1 - [2 - (2,4 - dichlorophenyl) - 1 - pentenyl] - 1*H* - imidazole and its acid addition salts.

9. 1 - [2 - (4 - chlorophenyl) - 3 - methyl - 1 - pentenyl] - 1*H* - imidazole and its acid addition salts.

10. 1 - [2 - (2,4 - dichlorophenyl) - 3 - methyl - 1 - pentenyl] - 1*H* - imidazole and its acid addition salts.

11. Pharmaceutical compositions, comprising as the active ingredient one or more ethenylimidazole derivatives of formula I given in claim 1 or a pharmaceutically acceptable acid addition salt thereof.

12. Phosphine-oxides of the formula

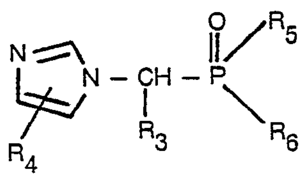

XI

in which $R_5$ and $R_6$ are the same or different and each represents an unsubstituted or substituted phenyl group, $R_3$ represents a hydrogen atom or an alkyl group with 1 to 6 carbon atoms and R4 is as defined in claim 1.

13. Phosphine-oxides of the formula given in claim 12, in which $R_5$ and $R_6$ are unsubstituted phenyl groups and $R_3$ and $R_4$ are as defined in claim 12.

**Claims for the Contracting State: AT**

1. Process for the preparation of ethenylimidazole derivatives of the formula

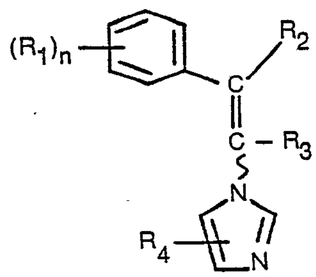

I

in which n is 0, 1 or 2, $R_1$ represents a halogen atom, a trifluoromethylgroup or an alkyl or alkoxy group with at most 6 carbon atoms, the two substituents being the same or different when n is 2, $R_2$ represents an alkyl group with 1 to 6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or an alkenyl group with 2 to 4 carbon atoms and $R_4$ represents a hydrogen atom or an alkyl group with 1 to 6 carbon atoms, and their acid addition salts, characterized in that an imidazole-ethanol of the formula

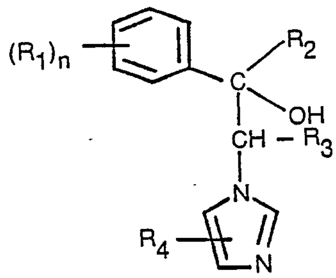

V

in which the R-symbols and n are as hereinbefore defined, is dehydrated.

2. Process according to claim 1, characterized in that dehydration is effected by treatment with a dehydrating agent such as thionyl chloride or hexamethylphosphortriamide.

3. Process for the preparation of ethenylimidazole derivatives as defined in claim 1, characterized in that a carboxylic acid of the formula

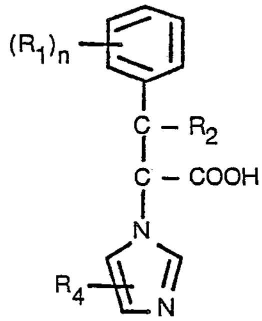

VII

in which the R-symbols and n are as defined in claim 1, is decarboxylated.

4. Process according to claim 3, characterized in that the compound of formula VII is heated at a temperature between 150 and 250°C.

5. Process for the preparation of ethenylimidazole derivatives of formula I, given in claim 1, in which $R_3$ represents a hydrogen atom or an alkyl group with 1 to 6 carbon atoms and the other R-symbols and n are as defined in claim 1, characterized in that a phosphine-oxide of the formula

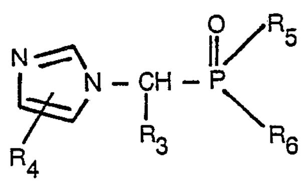

XI

in which $R_5$ and $R_6$ are the same or different and each represents an unsubstituted or substituted phenyl group and $R_3$ and $R_4$ are as defined above, is reacted with a ketone of the formula

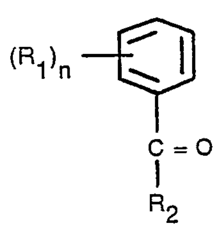

VIII

in which $R_1$, $R_2$ and n are as defined in claim 1, in the presence of an alkyl lithium and the product obtained is subsequently decomposed with water.

6. Process according to any one of claims 1—5, characterized in that in the compound of formula I, $R_1$ is chlorine, n is 1 or 2, $R_2$ is an alkyl group with 2—4 carbon atoms or cyclohexyl and $R_3$ and $R_4$ are hydrogen atoms.

7. Process according to any of claims 1—5, characterized in that in the compound of formula I $(R_1)_n$=4-chloro or 2,4-dichloro.

8. Process according to claim 6 or 7, characterized in that in the compound of formula I $R_2$ is n-propyl, *sec* butyl, *tert* butyl or cyclohexyl.

9. Process according to any of claims 1—8, characterized in that the compound prepared is selected from

1-[2-(2,4-dichlorophenyl)-3,3-dimethyl-1-butenyl]-1*H*-imidazole,
1-[2-cyclohexyl-2-(2,4-dichlorophenyl)ethenyl]-1*H*-imidazole,
1-[2-(4-chlorophenyl)-2-cyclohexylethenyl]-1*H*-imidazole,
1-[2-(2,4-dichlorophenyl)-1-pentenyl]-1*H*-imidazole,
1-[2-(4-chlorophenyl)-3-methyl-1-pentenyl]-1*H*-imidazole,
1-[2-(2,4-dichlorophenyl)-3-methyl-1-pentenyl]-1*H*-imidazole

and their acid addition salts.

10. Process for the preparation of phosphine-oxides of the formula

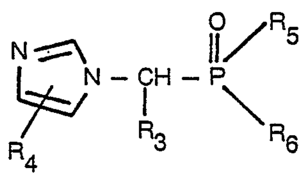

XI

in which the R-symbols are as defined in claim 5, characterized in that a diphenylphosphine-oxide of the formula

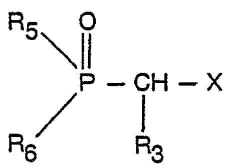

, XII

in which X is a chlorine or bromine atom and $R_3$, $R_5$ and $R_6$ are as hereinbefore defined, is reacted with an imidazole derivative of the formula

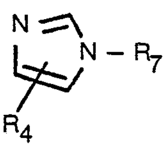

XIII

in which $R_7$ is a sodium, potassium or lithium atom.

**Patentansprüche für die Vertragsstaaten: BE CH DE FR GB IT LI LU NL SE**

1. Ethenylimidazol-Derivate der Formel

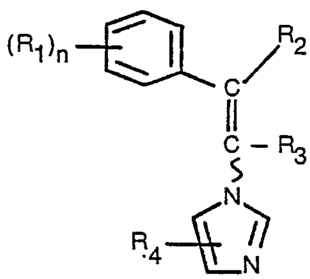

I

in der n 0, 1 oder 2 bedeutet, $R_1$ ein Halogenatom, eine Trifluormethylgruppe oder eine Alkyl- oder Alkoxygruppe mit höchstens 6 Kohlenstoffatomen darstellt, wobei die beiden Substituenten gleich oder verschieden sind, wenn n 2 ist, $R_2$ eine Alkylgruppe mit 1 bis 6 Kohlenstoffatomen, eine Alkenylgruppe mit 2 bis 4 Kohlenstoffatomen oder eine Cycloalkylgruppe mit 3 bis 6 Kohlenstoffatomen bedeutet, $R_3$ ein Wasserstoffatom, eine Alkylgruppe mit 1 bis 6 Kohlenstoffatomen oder eine Alkenylgruppe mit 2 bis 4 Kohlenstoffatomen darstellt, und $R_4$ ein Waserstoffatom oder eine Alkylgruppe mit 1 bis 6 Kohlenstoffatomen bedeutet, und deren Säureadditionssalze.

2. Ethenylimidazol-Derivate nach Anspruch 1, in welchen $R_1$ Chlor ist, n 1 oder 2 bedeutet, $R_2$ eine Alkylgruppe mit 2 bis 4 Kohlenstoffatomen oder Cyclohexyl ist und $R_3$ und $R_4$ Wasserstoffatome sind.

3. Ethenylimidazol-Derivate nach Anspruch 1, in welchen $(R_1)_n$ 4-Chlor oder 2,4-Dichlor bedeutet.

4. Ethenylimidazol-Derivate nach Anspruch 2 oder 3, in welchen $R_2$ n-Propyl, sec.-Butyl, tert.-Butyl oder Cyclohexyl ist.

5. 1-[2-(2,4-Dichlorphenyl)-3,3-dimethyl-1-butenyl]-1H-imidazol und dessen Säureadditionssalze.

6. 1-[2-Cyclohexyl-2-(2,4-dichlorphenyl)ethenyl]-1H-imidazol und dessen Säureadditionssalze.

7. 1-[2-(4-Chlorphenyl)-2-cyclohexylethenyl]-1H-imidazol und dessen Säureadditionssalze.

8. 1-[2-(2,4-Dichlorphenyl)-1-pentenyl]-1H-imidazol und dessen Säureadditionssalze.

9. 1-[2-(4-Chlorphenyl)-3-methyl-1-pentenyl]-1H-imidazol und dessen Säureadditionssalze.

10. 1-[2-(2,4-Dichlorphenyl)-3-methyl-1-pentenyl]-1H-imidazol und dessen Säureadditionssalze.

11. Pharmazeutische Mischungen, die als Wirkstoff ein oder mehrere Ethenylimidazol-Derivate der in Anspruch 1 angegebenen Formel I oder ein pharmazeutisch annehmbares Säureadditionssalz davon enthalten.

12. Phosphinoxide der Formel

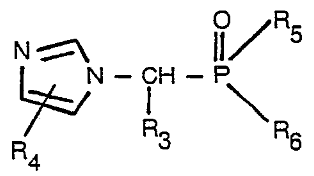

XI

in der $R_5$ und $R_6$ gleich oder verschieden sind und jeweils eine unsubstituierte oder substituierte Phenylgruppe bedeuten, $R_3$ ein Wasserstoffatom oder eine Alkylgruppe mit 1 bis 6 Kohlenstoffatomendarstellt und $R_4$ wie in Anspruch 1 definiert ist.

13. Phosphinoxide der in Anspruch 12 angegebenen Formel, in der $R_5$ und $R_6$ unsubstituierte Phenylgruppen sind und $R_3$ und $R_4$ wie in Anspruch 12 definiert sind.

**Patentansprüche für den Vertragsstaat: AT**

1. Verfahren zur Herstellung von Ethenylimidazol-Derivaten der Formel

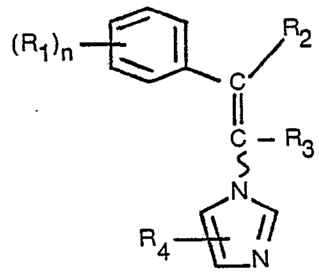

I

in der n 0, 1 oder 2 bedeutet, $R_1$ ein Halogenatom, eine Trifluormethylgruppe oder eine Alkyl- oder Alkoxygruppe mit höchstens 6 Kohlenstoffatomen darstellt, wobei die beiden Substituenten gleich oder verschieden sind, wenn n 2 ist, $R_2$ eine Alkylgruppe mit 1 bis 6 Kohlenstoffatomen, eine Alkenylgruppe mit 2 bis 4 Kohlenstoffatomen oder eine Cycloalkylgruppe mit 3 bis 6 Kohlenstoffatomen bedeutet, $R_3$ ein Wasserstoffatom, eine Alkylgruppe mit 1 bis 6 Kohlenstoffatomen oder eine Alkenylgruppe mit 2 bis 4 Kohlenstoffatomen darstellt, und $R_4$ ein Wasserstoffatom oder eine Alkylgruppe mit 1 bis 6 Kohlenstoffatomen bedeutet, und deren Säureadditionssalzen, dadurch gekennzeichnet, daß ein Imidazol-ethanol der Formel

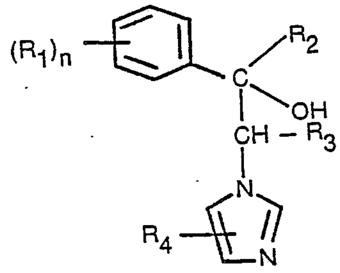

V

in der die R-Symbole und n wie vorhin angegeben definiert sind, dehydratisiert wird.

2. Verfahren nach Anspruch 1, dadurch gekennzeichnet, daß die Dehydratation durch Behandlung mit einem Dehydratisierungsmittel, wie Thionylchlorid oder Hexamethylphosphortriamid, bewirkt wird.

3. Verfahren zur Herstellung von Ethenylimidazol-Derivaten wie in Anspruch 1 definiert, dadurch gekennzeichnet, daß eine Carbonsäure der Formel

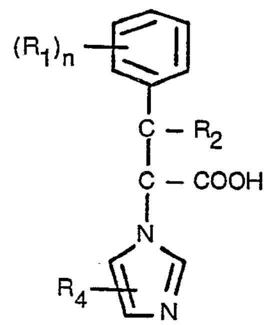

VII

in der die R-Symbole und n wie ion Anspruch 1 definiert sind, decarboxyliert wird.

4. Verfahren nach Anspruch 3, dadurch gekennzeichnet, daß die Verbindung der Formel VII auf eine Temperatur zwischen 150 und 250°C erhitzt wird.

5. Verfahren zur Herstellung von Ethenylimidazol-Derivaten der Formel I, angegeben in Anspruch 1, in der $R_3$ ein Wasserstoffatom oder eine Alkylgruppe mit 1 bis 6 Kohlenstoffatomen darstellt und die anderen R-Symbole und n wie in Anspruch 1 definiert sind, dadurch gekennzeichnet, daß ein Phosphinoxid der Formel

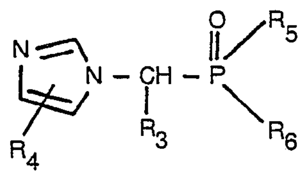

XI

in der $R_5$ und $R_6$ gleich oder verschieden sind und jeweils eine unsubstituierte oder substituierte Phenylgruppe darstellen und $R_3$ und $R_4$ wie oben definiert sind, mit einem Keton der Formel

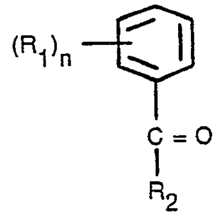

VIII

in der $R_1$, $R_2$ und n wie in Anspruch 1 definiert sind, in Gegenwart von einem Alkyllithium umgesetzt und das erhaltene Produkt anschließend mit Wasser zersetzt wird.

6. Verfahren nach einem der Ansprüche 1 bis 5, dadurch gekennzeichnet, daß in der Verbindung der Formel I $R_1$ Chlor bedeutet, n 1 oder 2 ist, $R_2$ eine Alkylgruppe mit 2 bis 4 Kohlenstoffatomen oder Cyclohexyl ist und $R_3$ und $R_4$ Wasserstoffatome sind.

7. Verfahren nach einem der Ansprüche 1 bis 5, dadurch gekennzeichnet, daß in der Verbindung der Formel I $(R_1)_n$ 4-Chlor oder 2,4-Dichlor bedeutet.

8. Verfahren nach Anspruch 6 oder 7, dadurch gekennzeichnet, daß in der Verbindung der Formel I $R_2$ n-Propyl, sec.-Butyl, tert.-Butyl oder Cyclohexyl ist.

9. Verfahren nach einem der Ansprüche 1 bis 8, dadurch gekennzeichnet, daß die hergestellte Verbindung ausgewählt is aus

1-[2-(2,4-Dichlorphenyl)-3,3-dimethyl-1-butenyl]-1H-imidazol,
1-[2-Cyclohexyl-2-(2,4-dichlorphenyl)ethenyl]-1H-imidazol,
1-[2-(4-Chlorphenyl)-2-cyclohexylethenyl]-1H-imidazol,
1-[2-(2,4-Dichlorphenyl)-1-pentenyl]-1H-imidazol,
1-[2-(4-Chlorphenyl)-3-methyl-1-pentenyl]-1H-imidazol,
1-[2-(2,4-Dichlorphenyl)-3-methyl-1-pentenyl]-1H-imidazol

und deren Säureadditionssalzen.

10. Verfahren zur Herstellung von Phosphinoxiden der Formel

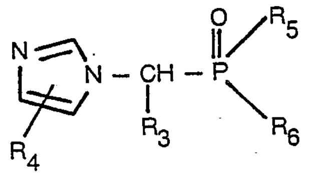

XI

in der die R-Symbole wie in Anspruch 5 definiert sind, dadurch gekennzeichnet, daß ein Diphenylphosphinoxid der Formel

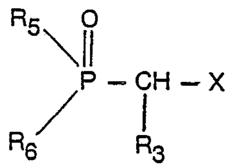

XII

in der X ein Chlor- oder Bromatom ist und $R_3$, $R_5$ und $R_6$ wie vorhin definiert sind, mit einem Imidazol-Derivat der Formel

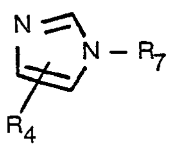

XIII

in der $R_7$ ein Natrium-, Kalium- oder Lithiumatom darstellt, umgesetzt wird.

**Revendications pour les Etats contractants: CH DE FR GB IT LI LU NL SE**

1. Dérivés d'éthénylimidazole de formule

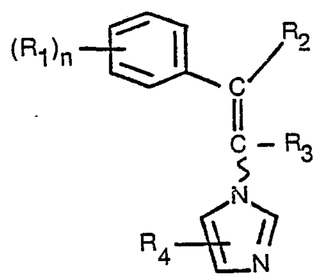

I

où n représente 0, 1 ou 2.

$R_1$ représente au atome d'halogène, un radical trifluorométhyle ou un radical alcoyle ou alcoxy de 6 atomes de carbone au maximum, les deux substituants étant identiques ou différents lorsque n représente 2,

$R_2$ représente un radical alcoyle de 1 à 6 atomes de carbone, un radical alcényle de 2 à 4 atomes de carbone ou un radical cycloalcoyle de 3 à 6 atomes de carbone,

$R_3$ représente un atome d'hydrogène, un radical alcoyle de 1 à 6 atomes de carbone ou un radical alcényle de 2 à 4 atomes de carbone et

$R_4$ représente un atome d'hydrogène, un radical alcoyle de 1 à 6 atomes de carbone, et leurs sels d'addition d'acides.

2. Dérivés d'éthénylimidazole suivant la revendication 1, dans lesquels $R_1$ représente le chlore, n représente 1 ou 2, $R_2$ représente un radical alcoyle de 2—4 atomes de carbone ou cyclohexyle et $R_3$ et $R_4$ représentent des atomes d'hydrogène.

3. Dérivés d'éthénylimidazole suivant la revendication 1, dans lesquels $(R_1)_n$ représente un radical 4-chloro ou 2,4-dichloro.

4. Dérivés d'éthénylimidazole suivant la revendication 2 ou 3, dans lesquels $R_2$ représente un radical n-propyle, s-butyle, t-butyle ou cyclohexyle.

5. Le 1-[2-(2,4-dichlorophényl)-3,3-diméthyl-1-butényl]-1*H*-imidazole et ses sels d'addition d'acides.

6. Le 1-[2-cyclohexyl-2-(2,4-dichlorophényl)éthényl]-1*H*-imidazole et ses sels d'addition d'acides.

7. Le 1-[2-(4-chlorophényl)-2-cyclohexyléthényl]-1*H*-imidazole et ses sels d'addition d'acides.

8. Le 1-[2-(2,4-dichlorophényl)-1-pentényl]-1*H*-imidazole et ses sels d'addition d'acides.

9. Le 1-[2-(4-chlorophényl)-3-méthyl-1-pentényl]-1*H*-imidazole et ses sels d'addition d'acides.

10. Le 1-[2-(2,4-dichlorophényl)-3-méthyl-1-pentényl)]-1*H*-imidazole et ses sels d'addition d'acides.

11. Compositions pharmaceutiques, comprenant comme constituant actif un ou plusieurs dérivés d'éthénylimidazole de formule I donnée dans la revendication 1 ou un sel d'addition d'acide pharmaceutiquement acceptable de ceux-ci.

12. Oxydes de phosphines de formule

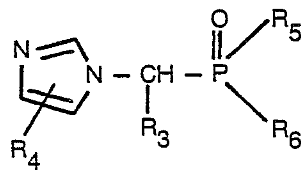

XI

où $R_5$ et $R_6$ sont identiques ou différents et représentent chacun un radical phényle non substitué ou substitué,

$R_3$ représente un atome d'hydrogène ou un radical alcoyle de 1 à 6 atomes de carbone et

$R_4$ est tel que défini dans la revendication 1.

13. Oxydes de phosphines de la formule donnée dans la revendication 12, où $R_5$ et $R_6$ représentent des radicaux phényle non substitués et $R_3$ et $R_4$ sont tels que définis dans la revendication 12.

**Revendications pour l'Etat contractant: AT**

1. Procédé de préparation de dérivés d'éthénylimidazole de formule

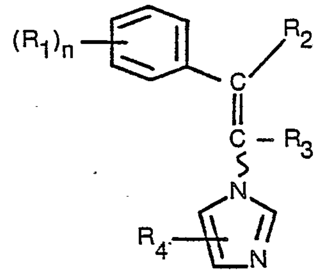

I

où n représente 0, 1 ou 2,

$R_1$ représente un atome d'halogène, un radical trifluoroéthyle ou un radical alcoyle ou alcoxy de 6 atomes de carbone au maximum, les deux substituants étant identiques ou différents lorsque n représente 2,

$R_2$ représente un radical alcoyle de 1 à 6 atomes de carbone, un radical alcényle de 2 à 4 atomes de carbone ou un radical cycloalcoyle de 3 à 6 atomes de carbone,

$R_3$ représente un atome d'hydrogène, un radical alcoyle de 1 à 6 atomes de carbone ou un radical alcényle de 2 à 4 atomes de carbone et

$R_4$ représente un atome d'hydrogène ou un radical alcoyle de 1 à 6 atomes de carbone,
et de leurs sels d'addition d'acides,
caractérisé en ce qu'un imidazole-éthanol de formule

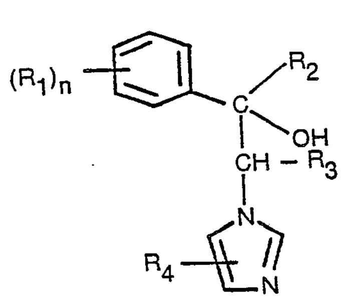

V

où les symboles R et n sont tels que définis ci-dessus est déshydraté.

2. Procédé suivant la revendication 1, caractérisé en ce que la déshydratation est effectuée par traitement au moyen d'un agent déshydratant tel que le chlorure de thionyle ou l'hexaméthylphosphorotriamide.

3. Procédé de préparation de dérivés d'éthénylimidazole tels que définis dans la revendication 1, caractérisé en ce qu'un acide carboxylique de formule

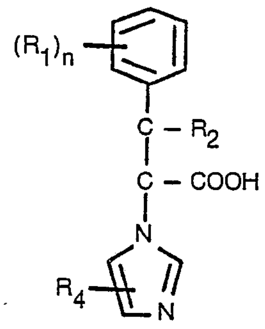

VII

où les symboles R et n sont tels que définis dans la revendication 1, est décarboxylé.

4. Procédé suivant la revendication 3, caractérisé en ce que le composé de formule VII est chauffé à une température entre 150 et 250°C.

5. Procédé de préparation de dérivés d'éthénylimidazole de formule I donnée dans la revendication 1, où $R_3$ représente un atome d'hydrogène ou un radical alcoyle de 1 à 6 atomes de carbone et les autres symboles R et n sont tels que définis dans la revendication 1, caractérisé en ce qu'un oxyde de phosphine de formule

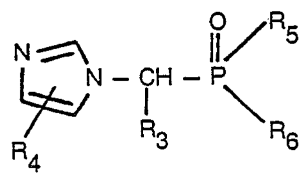

XI

où $R_5$ et $R_6$ sont identiques ou différents et représentent chacun un radical phényle non substitué ou substitué et $R_3$ et $R_4$ sont tels que définis ci-dessus, est mis à réagir avec une cétone de formule

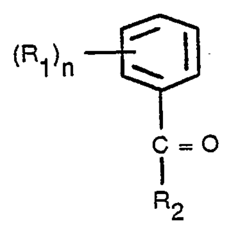

VIII

où $R_1$, $R_2$ et n sont tels que définis dans la revendication 1, en présence d'un alcoyllithium et le produit résultant est ensuite décomposé au moyen d'eau.

6. Procédé suivant l'une quelconque des revendications 1—5, caractérisé en ce que dans le composé de formule I, $R_1$ représente le chlore, n représente 1 ou 2, $R_2$ représente un radical alcoyle de 2—4 atomes de carbone ou cyclohexyle et $R_3$ et $R_4$ représentent des atomes d'hydrogène.

7. Procédé suivant l'une quelconque des revendications 1—5, caractérisé en ce que dans le composé de formule I, $(R_1)_n$ représente un radical 4-chloro ou 2,4-dichloro.

8. Procédé suivant la revendication 6 ou 7, caractérisé en ce que dans le composé de formule I, $R_2$ représente un radical n-propyle, s-butyle, t-butyle ou cyclohexyle.

9. Procédé suivant l'une quelconque des revendications 1—8, caractérisé en ce que le composé préparé est choisi parmi

le 1-[2-(2,4-dichlorophényl)-3,3-diméthyl-1-butényl]-1*H*-imidazole,
le 1-[2-cyclohexyl-2-(2,4-dichlorophényl)éthényl]-1*H*-imidazole,
le 1-[2-(4-chlorophényl)-2-cyclohexyléthényl]-1*H*-imidazole,
le 1-[2-(2,4-dichlorophényl)-1-pentényl]-1*H*-imidazole,
le 1-[2-(4-chlorophényl)-3-méthyl-1-pentényl]-1*H*-imidazole,
le 1-[2-(2,4-dichlorophényl)-3-méthyl-1-pentényl]-1*H*-imidazole,
et leurs sels d'addition d'acides.

10. Procédé de préparation d'oxydes de phosphines de formule

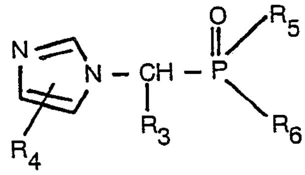

XI

où les symboles R sont tels que définis dans la revendication 5, caractérisé en ce qu'un oxyde de diphénylphosphine de formule

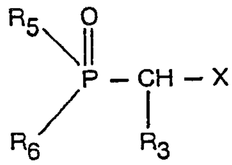

XII

où X représente un atome de chlore ou de brome et $R_3$, $R_5$ et $R_6$ sont tels définis ci-dessus, est mis à régir avec un dérivé d'imidazole de formule

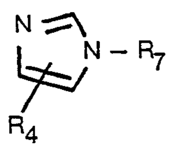

XIII

où $R_7$ représente un atome de sodium, de potassium ou de lithium.